(19) Europäisches Patentamt
European Patent Office
Office européen des brevets

(11) Publication number: **0 680 953 A1**

# (12) EUROPEAN PATENT APPLICATION

(21) Application number: **95106463.3**

(22) Date of filing: **02.05.95**

(51) Int. Cl.$^{6}$: **C07D 217/14**, A61K 31/47, C07D 401/06, C07D 217/16, C07D 405/06, C07D 217/02, C07D 491/04

(30) Priority: **03.05.94 HU 9401281**

(43) Date of publication of application: **08.11.95 Bulletin 95/45**

(84) Designated Contracting States: **AT BE CH DE ES GB GR LI NL SE**

(71) Applicant: **EGIS GYOGYSZERGYAR RT.**
**Kereszturi ut 30-38**
**H-1106 Budapest (HU)**

(72) Inventor: **Balogh, Gyula, Dr.**
**Deceaded**
**(HU)**
Inventor: **Doman, Imre**
**18/b Mohacs u.**
**H-1135 Budapest (HU)**
Inventor: **Blasko, Gabor, Dr.**
**21 Monar E. u.**
**H-1113 Budapest (HU)**
Inventor: **Simig, Gyula, Dr.**
**25 Hollosy S. u.**
**H-1126 Budapest (HU)**
Inventor: **Kovacs née Kaszab, Erzsébet**
**68 Noszlopy u.**
**H-1104 Budapest (HU)**
Inventor: **Gyertyan, Istvan, Dr.**
**33 Koronafürt u.**
**H-1165 Budapest (HU)**
Inventor: **Egyed, Andras, Dr.**
**58 Ujvidék u.**
**H-1145 Budapest (HU)**
Inventor: **Gacsalyi, Istvan, Dr.**
**64/b Tárogato u.**
**H-1021 Budapest (HU)**
Inventor: **Bilkei-Gorzo, Andras, Dr.**
**27 Körösi-Csoma S. u.**
**H-1105 Budapest (HU)**
Inventor: **Pallagi, Katalin**
**25 Hold u.**
**H-1054 Budapest (HU)**
Inventor: **Szemerédi, Katalin, Dr.**
**3 Vörösvari u.**
**H-1035 Budapest (HU)**
Inventor: **Kazo neé Daroczi, Klara, dipl. chem.**
**73 Szent Korona u.**
**H-1161 Budapest (HU)**

(74) Representative: **Beszédes, Stephan G., Dr.**
**Patentanwalt**
**Postfach 11 68**
**D-85201 Dachau (DE)**

(54) **1-[Styryl]- and 1-[2'-(heteroaryl)-ethenyl]-isoquinoline derivatives, a process for preparing them as well as medicaments containing them and the use of them.**

(57) A subject-matter of the invention are 1-[styryl]- and 1-[2'-(heteroaryl)-ethenyl]-isoquinoline derivatives having the formula

EP 0 680 953 A1

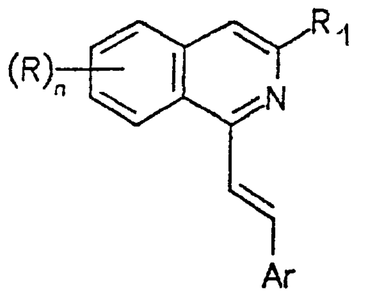

I )

wherein

n  is 1, 2, 3 or 4,

R  which represent(s) hydrogen, $C_{1-7}$-alkyl, $C_{1-7}$-alkoxy or hydroxy, or two substituents R form together a $C_{1-7}$-alkylenedioxy group attached to two adjacent ring carbon atoms,

$R_1$  represents hydrogen or $C_{1-7}$-alkyl and

Ar  stands for an aryl or heteroaryl group containing one or two oxygen, nitrogen and/or sulfur atom(s), optionally carrying one or more substituent(s) selected from halogen, trihalomethyl, hydroxy, $C_{1-7}$-alkyl, $C_{1-7}$-alkoxy, $C_{1-7}$-alkylenedioxy (attached to two adjacent ring carbon atoms), cyano, nitro, amino, mono- and di-($C_{1-7}$-alkyl)-amino,

as well as acid addition salts thereof.

Furthermore the invention is concerned with a process for preparing these compounds as well as medicaments containing them and the use both of the novel and of the known compounds for preparing medicaments having an anxiolytic effect.

The invention is concerned with novel 1-[styryl]- and 1-[2'-(heteroaryl)-ethenyl]-isoquinoline derivatives with useful pharmacological, particularly anxiolytic, effects, a process for preparing them as well as medicaments containing them and the use of them and of known 1-[styryl]- and 1-[2'-(heteroaryl)-ethenyl]-isoquinoline derivatives.

A great number of 1-(styryl)-isoquinoline derivatives and the pharmaceutical activities thereof has been known from the literature. Processes for the preparation of this group of compounds have also been described.

The synthesis routes well-known from the isoquinoline chemistry of a synthesis for the preparation of 6,7-di-[alkoxy]-1-[styryl]-isoquinolines exerting spasmolytic and analgetic effects are illustrated by the following reaction scheme.

**Reaction scheme**

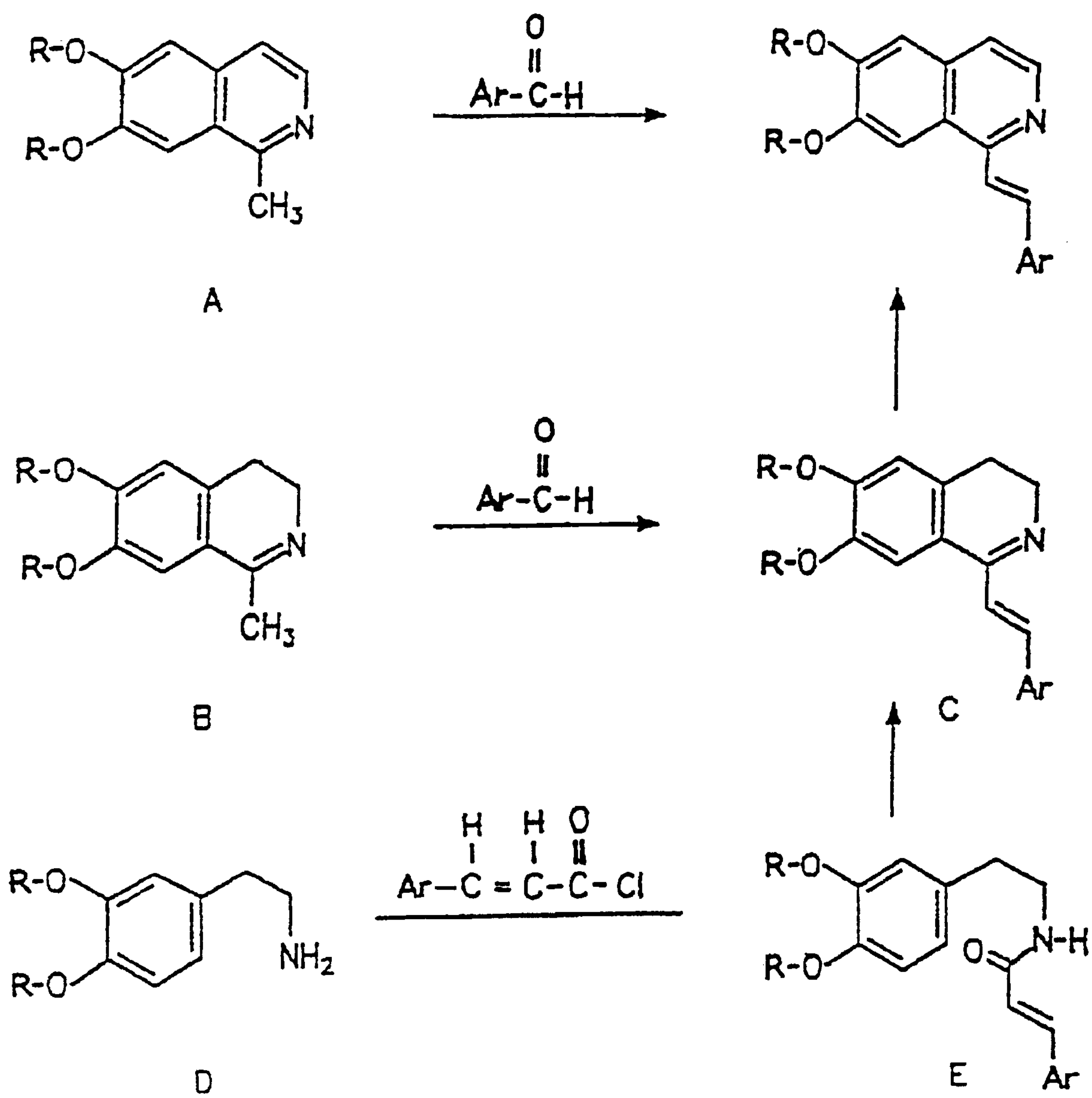

A

B

C

D

E

According to the known synthesis 6,7-di-[alkoxy]-1-[styryl]-isoquinolines can be prepared by condensing an appropriate 6,7-di-[alkoxy]-1-[methyl]-isoquinoline of general formula A with an aldehyde of general formula

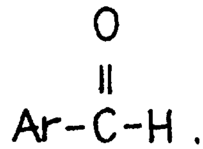

The condensation of the appropriate 6,7-di-[alkoxy]-1-[methyl]-3,4-di-[hydro]-isoquinoline of general formula B with the aldehyde of general formula

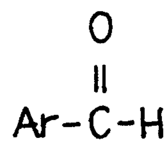

results in the corresponding 6,7-di-[alkoxy]-1-[styryl]-3,4-di(hydro)-isoquinoline of general formula C. The latter compounds can also be prepared by subjecting the {2-[3',4'-di-(alkoxy)-phen-1-yl]-eth-1-yl}-{cinnamoyl}-amines of general formula E obtained from the appropriate {2-[3',4'-di-(alkoxy)-phen-1-yl]-eth-1-yl)-amines of general formula D to a cyclization reaction of Bischler-Napieralski type. Furthermore, the 6,7-di-[alkoxy]-1-[styryl]--isoquinolines can be produced by a catalytic dehydrogenation of the corresponding 6,7-di-[alkoxy]-1-[styryl]-3,4-di-[hydro]-isoquinolines of general formula C.

According to published German patent application (Offenlegungsschrift) 1,902,402 6,7-di-[methoxy]-1-[styryl]-isoquinolines and 6,7-di-[methoxy]-1-[2'-(heteroaryl)-ethenyl]-isoquinolines are produced by the above process. It also has been mentioned that the said compounds inhibit the platelet functions (e.g. adhesion, agglomeration and retraction) and the liberation of biogenous amines from the amine-storing cells.

Some 1-[styryl]-isoquinoline derivatives can also be prepared from 2-[aryl]-2-[methoxy]-ethylamines, such as by acylation with styrylacetyl chloride, and a cyclization reaction of Bischler-Napieralski type (J. Knabe, H.D. Höltje: Arch. Pharm. 303 [1970], 404) to yield, for example, 6,7-di-[methoxy]-1-[3',4'-di-(methoxy)-styryl]-isoquinoline.No use of the compounds described there has been indicated.

The problem underlying to the invention is to create novel isoquinoline derivatives showing valuable pharmacological properties, particularly anxiolytic activity, and a process for preparing them as well as medicaments containing them and the use of them and of known 1-[styryl]-isoquinoline derivatives for a new field of pharmaceutical indication.

Surprisingly this has been solved by the invention.

Thus a subject-matter of the invention are 1-[styryl]and 1-[2'-(heteroaryl)-ethenyl]-isoquinoline derivatives having the formula

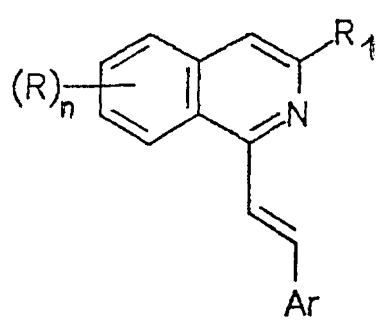

I ,

wherein

n is 1, 2, 3 or 4,

R which may be identical or different, represent(s) hydrogen, $C_{1-7}$-alkyl, $C_{1-7}$-alkoxy or hydroxy, or two substituents R form together a $C_{1-7}$-alkylenedioxy group; attached to two adjacent ring

carbon atoms,

$R_1$ represents hydrogen or $C_{1-7}$-alkyl and

Ar stands for an aryl or heteroaryl group containing one or two oxygen, nitrogen and/or sulfur atom(s), optionally carrying one or more identical or different substituent(s) selected from the group consisting of halogen, trihalomethyl, hydroxy, $C_{1-7}$-alkyl, $C_{1-7}$-alkoxy, $C_{1-7}$-alkylenedioxy (attached to two adjacent ring carbon atoms), cyano, nitro, amino, mono- and di-($C_{1-7}$-alkyl)-amino,

as well as acid addition salts thereof,

with the further provision that

6,7-di-[methoxy]-1-[3',4'-di-(methoxy)-styryl]-isoquinoline,
6,7-di-[methoxy]-1-[3',4'-(methylenedioxy)-styryl]-isoquinoline,
6,7-di-[methoxy]-1-[3',4',5'-tri-(methoxy)-styryl]-isoquinoline,
6,7-di-[methoxy]-1-[3'-(methoxy)-4'-(hydroxy)-styryl]-isoquinoline,
6,7-di-[methoxy]-1-[3'-(hydroxy)-4'-(methoxy)-styryl]-isoquinoline,
6,7-di-[methoxy]-1-[4'-(chloro)-styryl]-isoquinoline, 6,7-di-[methoxy]-1-[styryl]-isoquinoline,
6,7-[methylenedioxy]-1-[3',4'-di-(methoxy)-styryl]-isoquinoline,
6,7-[methylenedioxy]-1-[3',4'-(methylenedioxy)-styryl]-isoquinoline,
6,7-di-[methoxy]-3-[methyl]-1-[4'-(chloro)-styryl]-isoquinoline,
6,7-di-[methoxy]-3-[methyl]-1-[3',4'-di-(methoxy)-styryl]-isoquinoline,
6,7-di-[methoxy]-3-[methyl]-1-[3',4'-(methylenedioxy)-styryl]-isoquinoline,
6,7-di-[methoxy]-3-[methyl]-1-[2'-(pyrid-3''-yl)-ethenyl]-isoquinoline, and
6,7-di-[methoxy]-1-[2'-(pyrid-3''-yl)-ethenyl]-isoquinoline

as well as their acid addition salts

are excepted.

The term "$C_{1-7}$-alkyl" refers to straight-chained or branched saturated hydrocarbon groups, e.g. methyl, ethyl, n-propyl, isopropyl and n-butyl groups. The term "$C_{1-7}$-alkoxy" refers to alkyl ether groups, wherein the term "alkyl" corresponds to the above definition, e.g. methoxy, ethoxy, n-propoxy, isopropoxy and n-butoxy groups. The term "mono or di-($C_{1-7}$-alkyl)-amino" denotes amino groups substituted by one or two identical or different alkyl group(s) corresponding to the above definition, e.g. methylamino, ethylamino, dimethylamino, diethylamino, methylethylamino and diisopropylamino groups.

Preferably the alkyl group(s) which may be represented by R and/or $R_1$ and/or the alkoxy group(s) which may be represented by R and/or the alkyl and/or alkoxy group(s) by which the aryl or heteroaryl group which is represented by Ar may be substituted and/or the alkyl part(s) of the mono-and/or di-($C_{1-7}$-alkyl)-amino group(s) by which the aryl or heteroaryl group which is represented by Ar may be substituted is/are such having from 1 to 4, particularly 1 or 2, above all 1, carbon atom(s).

The term "$C_{1-7}$-alkylenedioxy group" refers to groups of the formula $-O-(CH_2)_m-O-$, wherein m is 1, 2, 3 or 4, e.g. methylenedioxy, ethylenedioxy and propylenedioxy groups.

It is preferred that the alkylenedioxy group (attached to two adjacent ring carbon atoms) which may be formed by two R and/or the alkylenedioxy group (attached to two adjacent ring carbon atoms) by which the aryl or heteroaryl group which is represented by Ar may be substituted is/are such having from 1 to 4, particularly 1 or 2, above all 1, carbon atom(s).

The term "halogen" and "halo", respectively, encompasses all the four halogen atoms, i.e. fluorine, chlorine, bromine and iodine.

It is preferred that the halogen atom(s) and/or the halogen atoms of the trihalomethyl group(s) by which the aryl or heteroaryl group which is represented by Ar may be substituted is/are [a] chlorine and/or fluorine atom(s), particularly the latter one(s). Hence preferred representatives of the "trihalomethyl groups" are the tri-(fluoro)-methyl and tri-(chloro)-methyl groups, particularly the trifluoromethyl group.

Moreover it is preferred that the aryl group which may be represented by Ar is a phenyl or naphthyl group.

The term "heteroaryl" encompasses both monocyclic and bicyclic heteroaryl groups containing one or two oxygen, nitrogen and/or sulfur atom(s), optionally substituted as above indicated.

Preferably the heteroaryl group which may be represented by Ar is a furyl, thienyl, pyrrolyl, pyridyl, pyrazinyl, pyridazinyl, oxazolyl, isoxazolyl, thiazolyl, isothiazolyl, pyrimidyl, quinolyl or isoquinolyl group. Particularly preferred as heteroaryl group is a pyridyl group, above all the pyrid-2-yl or pyrid-3-yl group, a furyl group, above all the fur-2-yl group, or a thienyl group, above all the thien-1-yl or thien-2-yl group.

A preferred group of the 1-[styryl]- and 1-[2'-(heteroaryl)-ethenyl]-isoquinoline derivatives according to the invention are those having the formula

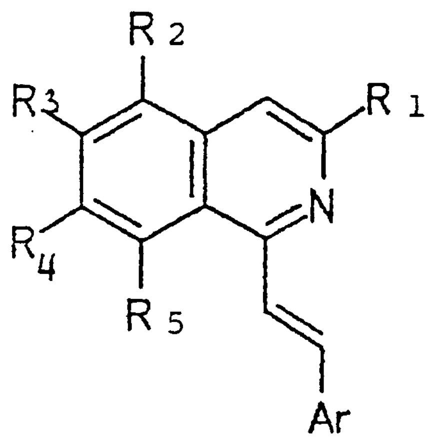

Ia ,

wherein

$R_1$ stands for hydrogen or $C_{1-7}$-alkyl,

$R_2$ represents hydrogen,

$R_3$ and $R_4$ each represent hydrogen or $C_{1-7}$-alkoxy or together form a $C_{1-7}$-alkylenedioxy group,

$R_5$ represents hydrogen or $C_{1-7}$-alkoxy and

Ar denotes phenyl, optionally carrying one or more identical or different substituent(s) selected from the group consisting of halogen, trihalomethyl, hydroxy, $C_{1-7}$-alkyl, $C_{1-7}$-alkoxy, $C_{1-7}$-alkylenedioxy (attached to two adjacent ring carbon atoms), cyano, nitro, amino, mono- and di-($C_{1-7}$-alkyl)-amino, or naphthyl, optionally carrying the above-mentioned substituents, or mono- or bicyclic heteroaryl containing one or two oxygen, nitrogen and/or sulfur atom(s) and, optionally carrying the above substituents

as well as their acid addition salts.

A particularly preferred group of 1-[styryl]- and 1-[2'-(heteroaryl)-ethenyl]-isoquinoline derivatives of formula Ia according to the invention are those in which

$R_1$ stands for hydrogen or methyl,

$R_2$ represents hydrogen,

$R_3$ and $R_4$ each represent methoxy or together form a methylenedioxy group (attached to two adjacent ring carbon atoms),

$R_5$ denotes hydrogen or methoxy and

Ar stands for phenyl, carrying a substituent selected from the group consisting of halogen, trifluoromethyl, nitro, methoxy, cyano and methylenedioxy (attached to two adjacent ring carbon atoms).

Preferably Ar represents phenyl, carrying a particularly single, substituent in position 4 or having one substituent each in the positions 3 and 4, particularly 3,4-di-(alkoxy)-phenyl, most particularly 3,4-di-(methoxy)-phenyl, or bearing methylenedioxy in positions 3 and 4.

The substituted phenyl group, which may be represented by Ar is preferably a 4-(trifluoromethyl)-phenyl, 4-(nitro)-phenyl, 4-(fluoro)-phenyl, 3,4-di-(methoxy)-phenyl, 4-(cyano)-phenyl or 4-(methoxy)-phenyl group, above all a 4-(trifluoromethyl)-phenyl, 4-(fluoro)-phenyl or 3,4-di-(methoxy)-phenyl group. Thus a most preferred group of 1-[styryl]- and 1-[2'-(heteroaryl)-ethenyl]-isoquinoline derivatives of formula Ia according to the invention are those, in which

$R_1$ and $R_2$ stand for hydrogen,

$R_3$ and $R_4$ each represent methoxy or together form a methylenedioxy group attached in positions 3 and 4,

$R_5$ denotes hydrogen and

Ar stands for 4-(trifluoromethyl)-phenyl, 4-(fluoro)-phenyl or 3,4-di-(methoxy)-phenyl.

Preferred 1-[styryl]- and 1-[2'-(heteroaryl)-ethenyl]-isoquinoline derivatives according to the invention are 6,7-[methylenedioxy]-1-[4'-(trifluoromethyl)-styryl]-isoquinoline, 1-[4'-(fluoro)-styryl]-6,7-di-[methoxy]-isoquinoline, 3-[methyl]-6,7-di-[methoxy]-1-[4'-(nitro)-styryl]-isoquinoline, 6,7-di-[methoxy]-1-[4'-(trifluoromethyl)-styryl]-isoquinoline, 1-[4'-(cyano)-styryl]-3-[methyl]-6,7,8-tri-[methoxy]-isoquinoline and 6,7-di-[methoxy]-1-[4'-(methoxy)-styryl]-isoquinoline as well as acid addition salts thereof.

From these particularly preferred are the first three and outstanding 6,7-[methylenedioxy]-1-[4'-(trifluoromethyl)-styryl]-isoquinoline as well as their salts.

Suitably the acid addition salts of the 1-[styryl]- and 1-[2'-(heteroaryl)-ethenyl]-isoquinoline derivatives according to the invention are pharmaceutically acceptable ones. They can be formed with inorganic or organic acids, e.g. hydrogen chloride, hydrogen bromide, nitric, sulfuric, phosphoric, maleic, fumaric, nicotinic, citric, malic, lactic, acetic, formic, mathanesulfonic and p-toluenesulfonic acid.

According to a further aspect of the invention there is provided for a process for prepare the 1-[styryl]- and 1-[2'-(heteroaryl)-ethenyl]-isoquinoline derivatives according to the invention, which are characterized in that in a manner known per se 1-[methyl]-isoquinoline derivatives of general formula

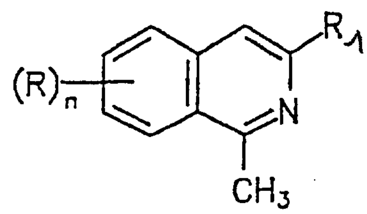

II,

wherein

n, R and $R_1$ are as above defined, are reacted with aldehydes of general formula

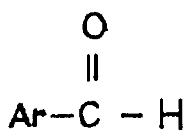

III,

wherein Ar is as above defined, in the presence of a condensing agent or an acidic catalyst and optionally 1-[styryl]- and 1-[2'- (heteroaryl)-ethenyl]-isoquinoline derivatives of general formula I thus obtained are converted into acid addition salts thereof or acid addition salts of 1-[styryl]- and 1-[2'-(heteroaryl)-ethenyl]-isoquinoline derivatives of general formula I thus obtained are converted into the free isoquinoline derivative bases of general formula I and/or into other acid addition salts.

For preparing specifically the 1-[styryl]- and 1-[2'-(heteroaryl)-ethenyl]-isoquinoline derivatives of formula Ia according to the invention as 1-[methyl]-isoquinoline derivative of general formula II such of general formula

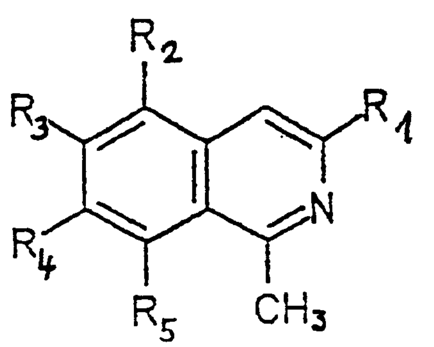

IIa,

wherein

$R_1$ $R_2$, $R_3$, $R_4$ and $R_5$ are as above defined, are reacted with the aldehydes of general formula III, in which Ar is as above defined.

According to a preferred embodiment as condensing agent(s) zinc chloride, phosphorus pentoxide, acetic anhydride and/or propionic anhydride is/are used. Conveniently the reaction is carried out at a temperature of from 60 to 180 °C, preferably 150 to 160 °C. The reaction time may be varied from 1 hour and 8 hours. Generally 0,5 to 2 mole(s), preferably 1,05 to 1,2 moles, of the aldehyde of general formula III is/are used for 1 mole of the 1- [methyl]-isoquinoline derivative of general formula II. The reaction mixture can be worked up by methods known per se. It can be preferable to cool the reaction mixture, particularly to a temperature of from 0 to 10 °C, to precipitate the desired 1-[styryl]- or 1-[2'-(heteroaryl)-ethenyl]-isoquinoline derivative with an appropriate solvent, e.g. an ester, such as ethyl acetate, or an ether, such as

diethylether, and to filter, wash and dry the separated product.

As already mentioned, according to a further preferred embodiment one can also proceed by carrying out the condensation of the compounds of general formulae II and III in the presence of an acidic catalyst. For this purpose preferably [a] $C_{1-7}$-monocarboxylic acid(s), particularly $C_{1-4}$-monocarboxylic acid(s), above all acetic acid, is/are used. Conveniently the reaction is carried out at a temperature of from 60 to 150 °C, preferably at the boiling point of the reaction mixture. The reaction time may be varied from 1 hour to 8 hours. Generally 0,5 to 2 mole(s), preferably 1,05 to 1,2 moles, of the aldehyde of general formula III is/are used for 1 mole of the 1-[methyl]-isoquinoline derivative of general formula II. The reaction mixture can be worked up by methods known per se. It can be preferable to cool the reaction mixture, add an appropriate solvent, e.g. a ketone, such as acetone, to it and make the mixture strongly acidic. In such cases the separating salt is isolated, e.g. by filtration, then it is washed and dried.

The optional conversion of the 1-[styryl]- and 1-[2'-(heteroaryl)-ethenyl]-isoquinoline derivatives of formula I according to the invention thus obtained into acid addition salts advantageously can be carried out by known methods, reacting the 1-[styryl]- and 1-[2'-(heteroaryl)-ethenyl]-isoquinoline derivatives of general formula I with an appropriate acid. Also the optional liberation of the 1-[styryl]- and 1-[2'-(heteroaryl)-ethenyl]-isoquinoline derivatives of general formula I from salts thereof advantageously can be carried out by methods known per se, such as by reacting the appropriate salt with a, preferably inorganic, base, e.g. alkali hydroxide, such as sodium hydroxide or potassium hydroxide.

As already mentioned, the 1-[styryl]- and 1-[2'-(heteroaryl)-ethenyl]-isoquinoline derivatives according to the invention or used according to the invention, respectively, possess valuable pharmacological, particularly anxiolytic, properties. This recognition is surprising, since such an activity so far has not been attributed to the known 1-[styryl]-isoquinoline derivatives in the literature. It has been found that the 1-[styryl]-and 1-[2'-(heteroaryl)-ethenyl]-isoquinoline derivatives according to the invention exhibit an outstandingly strong anxiolytic activity, which surpasses by orders of magnitude that of the hitherto known therapeutical agents. A further advantage of these compounds is that they are only slightly toxic.

Hence by the invention also there are provided for medicamants which are characterized in that they contain as [an] active ingredient(s) at least one compound according to the invention, suitably in admixture with at least one suitable inert solid and/or liquid pharmaceutical carrier and/or diluent and/or excipient.

Also the known 1-[styryl]-isoquinoline derivatives of formula I excluded above have an outstandingly strong anxiolytic activity with slight toxicity which is likewise surprising in view of that, as already mentioned, such an activity so far has not been attributed to these compounds. Hence a further subject-matter of the invention is the use of the compounds according to the invention and/or of 6,7-di-[methoxy]-1-[3',4'-di-(methoxy)-styryl]-isoquinoline, 6,7-di-[methoxy]-1-[3',4'-(methylenedioxy)-styryl]-isoquinoline, 6,7-di-[methoxy]-1-[3',4',5'-tri-(methoxy)-styryl]-isoquinoline, 6,7-di-[methoxy]-1-[3'-(methoxy)-4'-(hydroxy)-styryl]-isoquinoline,6,7-di-[methoxy]-1-[3'-(hydroxy)-4'-(methoxy)-styryl]-isoquinoline,6,7-di-[methoxy]-1-[4'-(chloro)-styryl]-isoquinoline, 6,7-di-[methoxy]-1-[styryl]-isoquinoline, 6,7-[methylenedioxy]-1-[3',4'-di-(methoxy)-styryl]-isoquinoline, 6,7-[methylenedioxy]-1-[3',4'-(methylenedioxy)-styryl]-isoquinoline, 6,7-di-[methoxy]-3-[methyl]-1-[4'-(chloro)-styryl]-isoquinoline, 6,7-di-[methoxy]-3-[methyl]-1-[3',4'-di-(methoxy)-styryl]-isoquinoline, 6,7-di-[methoxy]-3-[methyl]-1-[3',4'-(methylenedioxy)-styryl]-isoquinoline and/or 6,7-di-[methoxy]-1-[2'-(pyrid-3''-yl)-ethenyl]-isoquinoline and/or of their acid addition salts for preparing medicaments having an anxiolytic effect.

From the known 1-[styryl]-isoquinoline derivatives the use of 1-[3',4'-di-(methoxy)-styryl]-6,7-[methylenedioxy]-isoquinoline is preferred.

The activity of 1-[styryl]-isoquinoline derivatives of general formula I is shown by the following tests:

I. Acute toxicity

White mice belonging to the NMRI strain (body weight 20 to 25 g, both male and female) were used, 6 animals for each dose. The test compound was administered orally in a volume of 20 ml/kg, the maximum oral and intraperioneal doses were 1 000 mg/kg and 300 mg/kg, respectively. After treatment the animals were observed for a period of 7 days. The mice were kept under usual laboratory conditions. The toxicity data are summarized in the following Table I.

## Table I

| Compound | | $LD_{50}$ (mg/kg, i.p.) | $LD_{50}$ (mg/kg, p.o.) |
|---|---|---|---|
| Designation | No. of Example | | |
| 1-[4'-(Chloro)-styryl]-3-[methyl]-6,7-di-[methoxy]-isoquinoline | 2 | > 300 | > 1000 |
| 6,7-[Methylenedioxy]-1-[4'-(trifluoromethyl)--styryl]-isoquinoline | 3 | > 300 | > 1000 |
| 3-[Methyl]-6,7-di-[methoxy]-1-[4'-(nitro)--styryl]-isoquinoline | 4 | > 300 | > 1000 |
| 1-[3'-(Bromo)-styryl]-3-[methyl]-6,7--[methylenedioxy]-isoquinoline | 19 | > 300 | > 1000 |
| 1-[4'-(Fluoro)-styryl]-6,7-di-[methoxy]--isoquinoline | 5 | > 300 | > 1000 |
| 6,7-Di-[methoxy]-1-[4'-(trifluoromethyl)--styryl]-isoquinoline | 6 | > 300 | > 1000 |
| 1-[3',4'-Di-(methoxy)-styryl]-6,7-di--[methoxy]-isoquinoline | 7 | > 300 | 1000 |
| 1-[3',4'-Di-(methoxy)-styryl]-6,7--[methylenedioxy]-isoquinoline | 20 | > 300 | > 1000 |

Table I continued

| Compound | | | |
|---|---|---|---|
| Designation | No. of Example | $LD_{50}$ (mg/kg, i.p.) | $LD_{50}$ (mg/kg, p.o.) |
| 6,7-Di-[methoxy]-1-[4'-(methoxy)-styryl]--isoquinoline | 2 | > 300 | > 1000 |
| 1-[2',4'-Di-(chloro)-styryl]-isoquinoline | 8 | > 300 | > 1000 |
| 1-[4'-(Cyano)-styryl]-3-[methyl]-6,7,8-tri--[methoxy]-isoquinoline | 9 | > 300 | > 1000 |
| 7-(Chloro))-1-(methyl)-5-(phenyl)-1H-1,4--benzodiazepin-2(3H)-one [Diazepam] | Reference substance | > 300 | $780^{+}$ |

+ literary data

II. Anxiolytic activity

1. Vogel test (lick conflict test)

Male Wistar rats of 160 to 180 g body weight were kept free of food and drinking water for 24 and 48 hours, respectively. The test and carrier substances (0.4% by weight aqueous methylcellulose solution) were administered orally one hour before testing. Groups consisting of 8 animals were used. The rats within the experimental chamber were provided with drinking water through an inserted tube. After the animals' each twenty lapping for water the device emitted a 1.4 mA intensity electric shock through the drinking tube. During 5 minutes the shocks tolerated by the animals in order to quench their thirst were counted. The effect of treatment was expressed as the % increase of the tolerated shocks. The minimum effective dose (MED) was determined for each test compound (Vogel, J.R., Beer, B., Clody, D. E.: Psychopharmacologia (Berl.) 21, 1 [1971]. The data obtained are summarized in the following Table II.

Table II

| Compound | | Minimum effective dose (mg/kg, p.o.) |
|---|---|---|
| Designation | No. of Example | |
| 6,7-[Methylenedioxy]-1-[4'-(trifluoromethyl)-styryl]-isoquinoline | 3 | 0.00001 |
| 3-[Methyl]-6,7-di-[methoxy]-1-[4'-(nitro)-styryl]-isoquinoline | 4 | 0.01 |
| 1-[4'-(Fluoro)-styryl]-6,7-di-[methoxy]-isoquinoline | 5 | 0.0003 |
| 6,7-Di-[methoxy]-1-[4'-(trifluoromethyl)-styryl]-isoquinoline | 6 | 0.01 |
| 1-[3',4'-Di-(methoxy)-styryl]-6,7-[methylenedioxy]-isoquinoline | 20 | 0.001 |
| 6,7-Di-[methoxy]-1-[4'-(methoxy)-styryl]-isoquinoline | 21 | 0.03 |
| 1-[4'-(Cyano)-styryl]-3-[methyl]-6,7,8-tri-[methoxy]-isoquinoline | 9 | 0.01 |
| 7-(Chloro))-1-(methyl)-5-(phenyl)-1H-1,4-benzodiazepin-2(3H)-one [Diazepam] | Reference substance | 0.1 |

As it can be seen from the above Table II, the anxiolytic activity of the 1-[styryl]-isoquinoline derivatives according to the invention or used according to the invention, respectively, is stronger by orders of magnitude than that of 7-(chloro)-1-(methyl)-5-(phenyl)-1H-1,4-benzodiazepin-2(3H)-one[diazepam] used as reference substance.

2. Elevated plus maze test on rat

The test was carried out with the aid of a wooden plus-shaped maze elevated to a height of 50 cm. Two arms (100 cm long and 15 cm wide) - opposite to one another - were enclosed up to a height of 40 cm along their longer sides and at the end. The other two arms were without walls (open arms). The central

15x15 cm part was open. Male rats belonging to the Sprague Dawley strain and weighing 220 to 260 g were used as test animals. After pretreatment lasting 60 minutes the animals were placed into the central part of the maze. During the 5 minutes observation period the following parameters were recorded:

- time spent on the open arms
- time spent on the closed arms
- number of open arm entries
- number of closed arm entries.

The drug effect was expressed as percent increase of the time spent on the open arms and number of open arm entries. The minimum effective dose (MED) which caused a significant increase of the time spent on the open arms was calculated for every substance (Pelow, S., Chopin, P., File, S.E., Briley, M.: J. Neurosci. Methods 14 [1985], 149 to 167). The data are shown in the following Table III.

Table III

| Compound | | Minimum effective dose (mg/kg, p.o.) |
|---|---|---|
| Designation | No. of Example | |
| 6,7-[Methylenedioxy]-1-[4'-(trifluoromethyl)-styryl]-isoquinoline | 3 | 0.1 |
| 7-(Chloro)-1-(methyl)-5-(phenyl)-1H-1,4-benzodiazepin-2(3H)-one [Diazepam] | Reference substance | 2.5 |

From the above Table III it results that the 6,7-[methylenedioxy]-1-[4'-(trifluoromethyl)-styryl]-isoquinoline according to Example 3 proved to be 25 times more effective than the reference substance 7-(chloro))-1-(methyl)-5-(phenyl)-1H-1,4-benzodiazepin-2(3H)-one [diazepam].

3. Hole-board test

The experiment was carried out according to a slightly modified method of File (File, S. E., Wardill, A. G., Psychopharmacologia, 44 [1975], 53 to 59). Male Wistar rats (Charles River) weighing 160 to 200 g were fasted for 24 hours prior to the experiment. They were treated orally in a volume of 5 ml/kg one hour before testing, a treated group consisted of 12 animals. The test compounds were suspended in 0.4% by weight aqueous methylcellulose solution, the control group was treated with 0.4% by weight aqueous methylcellulose solution. All procedures were carried out in a quiet, air-conditioned laboratory between 9:00 and 13:00 hours at ambient temperature (23 + 2 °C). Locomotor and head-dipping activity were measured using automated test chambers (Digiscan, OMNITECH® Electronics Inc., Columbus, Ohio). The floor of each box contained 4 equally spaced holes (3.5 cm in diameter) through which rat could pop its head. Interruptions of infrared beams (16 of them is located 1.0 cm below and an other 163.0 cm above the floor level) were automatically recorded by Digiscan® analyser and then transmitted to a computer and analysed by OASIS® software. The hole-board testing involved placing a rat in the center of the floor and allowing it to explore for 5 minutes. At the end of each trial any boluses were removed and the box was thoroughly wiped. Minimum effective doses (MED) of the compounds were calculated from the head-dipping activity values. The results are shown in the following Table IV.

Table IV

| Compound | | Minimum effective dose (mg/kg, p.o.) |
|---|---|---|
| Designation | No. of Example | |
| 6,7-[Methylenedioxy]-1-[4'-(trifluoromethyl)-styryl]-isoquinoline | 3 | 0.0001 |
| 6,7-Di-[methoxy]-1-[4'-(trifluoromethyl)-styryl]-isoquinoline | 6 | 0.0001 |
| 6,7-Di-[methoxy]-1-[4'(methoxy)-styryl]-isoquinoline | 21 | 0.01 |
| 7-(Chloro))-1-(methyl)-5-(phenyl)-1H-1,4-benzodiazepin-2(3H)-one [Diazepam] | Reference substance | 0.1 |

From the above Table IV it results that all the three test compounds according to the invention proved to be more active than the reference substance 7-(chloro)-1-(methyl)-5-(phenyl)-1H-1,4-benzodiazepin-2-(3H)-one [diazepam], one by one order of magnitude and two by three orders of magnitude.

The medicaments according to the invention can be in the form of pharmaceutical preparations. These can be prepared by methods known per se and thus suitably by admixing the 1 or more 1-[styryl]- and 1-[2'-(heteroaryl)-ethenyl]-isoquinoline derivatives with 1 or more inert solid and/or liquid carrier (s) and/or diluent(s) and/or excipient(s) and bringing the mixture to a galenic form.

Advantageously the pharmaceutical preparations of the present invention are for oral administration, e.g. in form of tablets, pills, coated pills, dragées, solid or soft gelatin capsules, solutions, emulsions or suspensions, for parenteral administration, e.g. in form of injection solutions, or for rectal application, e.g. in form of suppositories.

As carrier(s) in tablets, coated tablets, dragées and solid gelatin capsules, for example, lactose, corn starch, potato starch, talc, magnesium carbonate, magnesium stearate, calcium carbonate, stearic acid and/or 1 or more salt(s) thereof can be contained. As carrier(s) in soft gelatin capsules, for example, 1 or more vegetable oil(s), fat(s), wax(es) and/or polyol(s) of suitable consistency can be contained. As carrier(s) in solutions and syrups, for example, water, 1 or are polyol(s) [polyethylene glycol(s)], saccharose and/or glucose can be contained. The injection solutions can comprise, for example, water, 1 or more alcohol(s), polyol(s), glycerol and/or 1 or more vegetable oil(s) as carrier(s). The suppositories can contain as carrier(s), for example, 1 or more oil(s), wax(es), fat(s) and/or polyol(s) of suitable consistency.

In addition, the pharmaceutical preparations of the invention may comprise 1 or more auxiliary agent(s) usually applied in the pharmaceutical technique, for example, 1 or more wetting and/or sweetening agent(s), aroma substance(s), salt(s) causing the change of osmotic pressure and/or buffer(s). The pharmaceutical preparations of the invention may further comprise 1 or more other active ingredient(s), too.

The daily dose of the 1-[styryl]- and 1-[2'-(heteroaryl)-ethenyl]-isoquinoline derivatives of general formula I can vary within wide ranges depending on several factors, e.g. on the activity of the active ingredient(s), the patient's condition and age and the severity of the disease. The preferred oral dose is generally 0.1 to 200 mg/day. It has to be stressed that the above dose is only of informative character and the administered dose must always be determined by the physician therapeutist.

The compounds of general formula I and their acid addition salts are to be administered to the patients in effective amounts.

The invention is further illustrated by the following Examples. The ratios of the components of the mixtures for crystallization, also as regards the indication of the melting points, are by volume.

I) Method A

<u>Example 1</u>

1-[2',6'-Di-(chloro)-styryl]-6,7-di-[methoxy]-isoquinoline

A mixture of 20.4 g (0.1 mole) of 1-[methyl]-6,7-di-[methoxy]-isoquinoline,18.9 g (0.108 mole) of 2,6-di-(chloro)-benzaldehyde and 15 ml of acetic anhydride is stirred in an oil bath of 150 to 160 °C for 3 hours. The termination of the reaction is controlled by thin layer chromatography. The reaction mixture is cooled to

a temperature of from 5 to 10 °C under stirring, 30 ml of ether are added to it, the separated crystals are filtered and washed twice with cold ether. The thus obtained raw product is recrystallized from a mixture of 600 ml of acetone and 130 ml of water. The clarified solution is stirred at a temperature of from 0 to 5 °C for 2 hours, the separated substance is filtered, washed with 25 ml of a 6:1.3 mixture of acetone and water of 0 °C and dried. Thus 28.4 g (79%) of 1-[2',6'-di-(chloro)-styryl]-6,7-di-[methoxy]-isoquinoline are obtained.
M.p.: 145 to 146 °C (acetone)

| Analysis for the formula $C_{19}H_{15}Cl_2NO_2$ (360.25): | | | | | | | | |
|---|---|---|---|---|---|---|---|---|
| Calculated:<br>Found: | C: | 63.35<br>62.87 | H: | 4.20<br>3.99 | N: | 3.89<br>3.95 | Cl: | 19.68 %<br>19.53 % |

$^1$H-NMR($CDCl_3$): δ 8.47 (1H, d, J = 5.5), 7.98 (1H, d, J = 16.0), 7.89 (1H, d, J = 16.0), 7.46 (1H, s), 7.43 (1H, d, J = 5.5), 7.37 (2H, d, J = 8.0), 7.12 (1H, m), 7.03 (1H, s), 4.02 (3H, s), 4.00 (3H, s).

Example 2

1-[4'-(Chloro)-styryl]-3-[methyl]-6,7-di-[methoxy]-isoquinoline

According to the method of Example 1 1-[4'-(chloro)-styryl]-3-[methyl]-6,7-di-[methoxy]-isoquinoline is prepared from 1,3-di-[methyl]-6,7-di-[methoxy]-isoquinoline with 4-(chloro)-benzaldehyde. Yield: 51%.
M.p.: 181 to 183 °C (ethyl acetate)

| Analysis for the formula $C_{20}H_{18}ClN0_2$ (339.83): | | | | | | | | |
|---|---|---|---|---|---|---|---|---|
| Calculated:<br>Found: | C: | 70.69<br>70.96 | H: | 5.34<br>5.33 | N: | 4.12<br>4.31 | Cl: | 10.43%<br>10.41% |

$^1$H-NMR ($CDCl_3$): δ 7.86 (1H, d, J = 15.5), 7.73 (1H, d, J = 15.5), 7.56 (2H, d, J = 8.5), 7.38 (1H, s), 7.34 (2H, d, J = 8.5), 7.23 (1H, s), 6.92 (1H, s), 4.02 (3H, s), 3.98 (3H, s), 2.66 (3H, s).

Example 3

6,7-[Methylenedioxy]-1-[4'-(trifluoromethyl)-styryl]-isoquinoline

According to the method of Example 1 6,7-[methylenedioxy]-1-[4'-(trifluoromethyl)-styryl]-isoquinoline is prepared from 1-[methyl]-6,7-[methylenedioxy]-isoquinoline with 4-(trifluoromethyl)-benzalhyde. Yield: 66%.
M.p.: 143 to 145 °C (methanol)

| Analysis for the formula $C_{19}H_{12}F_3N0_2$ (343.31): | | | | | | |
|---|---|---|---|---|---|---|
| Calculated:<br>Found: | C: | 66.47<br>65.80 | H: | 3.52<br>3.51 | N: | 4.08%<br>4.16% |

$^1$H-NMR ($CDCl_3$): δ 8.40 (1H, d, J = 5.5), 7.92 (1H, d, J = 15.6), 7.81 (1H, d, J = 15.6), 7.72 (2H, d, J = 8.3), 7.63 (2H, d, J = 8.3), 7.53 (1H, s), 7.40 (1H, d, J = 5.5), 7.04 (1H, s), 6.09 (2H, s).

Example 4

3-[Methyl]-6,7-di-[methoxy]-1-[4'-(nitro)-styryl]-isoquinoline

According to the method of Example 1 3-[methyl]-6,7-di-[methoxy]-1-[4'-(nitro)-styryl]-isoquinoline is prepared from 1,3-di-[methyl]-6,7-di-[methoxy]-isoquinoline with 4-(nitro)-benzalhyde. Yield: 64%.
M.p.: 231 to 232 °C (acetone : dioxane = 2 : 1)

| Analysis for the formula $C_{20}H_{18}N_2O_4$ (350.38): | | | | | | |
|---|---|---|---|---|---|---|
| Calculated:<br>Found: | C: | 68.56<br>68.96 | H: | 5.18<br>5.17 | N: | 8.00%<br>8.09% |

$^1$H-NMR ($CDCl_3$): δ 8.26 (2H, d, J=9.0), 8.00 (1H, d, J=15.6), 7.93 (1H, d, J=15.6), 7.78 (2H, d, J=9.0), 7.43 (1H, s), 7.34 (1H, s), 7.00 (1H, s), 4.07 (3H, s), 4.03 (3H, s), 2.69 (3H, s).

Example 5

1-[4'-(Fluoro)-styryl]-6,7-di-[methoxy]-isoquinoline

According to the method of Example 1 1-[4'-(fluoro)-styryl]-6,7-di-[methoxy]-isoquinoline is prepared from 1-[methyl]-6,7-di-[methoxy]-isoquinoline with 4-(fluoro)-benzaldehyde.
Yield: 51%.
M.p.: 169 to 171 °C (acetone)

| Analysis for the formula $C_{19}H_{16}FNO_2$ (309.35): | | | | | | |
|---|---|---|---|---|---|---|
| Calculated:<br>Found: | C: | 73.77<br>72.91 | H: | 5.21<br>4.92 | N: | 4.53%<br>4.75% |

$^1$H-NMR ($CDCl_3$) : δ 8.42 (1H, d, J=5.5), 7.90 (1H, d, J=15.5), 7.89 (1H, d, J=15.5), 7.67 (2H, m), 7.44 (1H, s), 7.40 (1H, d, J=5.5), 7.08 (2H, m), 7.02 (1H, s), 4.05 (3H, s), 4.01 (3H, s).

Example 6

6,7-Di-[methoxy]-1-[4'-(trifluoromethyl)-styryl]-isoquinoline

According to the method of Example 1 6,7-di-[methoxy]-1-[4'-(trifluoromethyl)-styryl]-isoquinoline is prepared from 1-[methyl]-6,7-di-[methoxy]-isoquinoline with 4-(trifluoromethyl)-benzalhyde. Yield: 57%.
M.p.: 146 to 148 °C (acetone)

| Analysis for the formula $C_{20}H_{16}F_3NO_2$ (359.36): | | | | | | |
|---|---|---|---|---|---|---|
| Calculated:<br>Found: | C: | 66.85<br>65.16 | H: | 4.49<br>5.01 | N: | 3.90%<br>3.73% |

$^1$H-NMR ($CDCl_3$): δ 8.44 (1H, d, J=5.6), 7.95 (1H, d, J=15.5), 7.86 (1H, d, J=15.5), 7.73 (2H, d, J=8.3), 7.63 (2H, d, J=8.3), 7.44 (1H, d, J=5.6), 7.43 (1H, s), 7.04 (1H, s), 4.06 (3H, s), 4.01 (3H, s).

Example 7

1-[3',4'-Di-(methoxy)-styryl]-6,7-di-[methoxy]-isoquinoline

According to the method of Example 1 1-[3',4'-di-(methoxy)-styryl]-6,7-di-[methoxy]-isoquinoline is prepared from 1-[methyl]-6,7-di-[methoxy]-isoquinoline with 3,4-di-(methoxy)-benzalhyde. Yield: 54%.
M.p.: 136 to 138 °C (acetone)

| Analysis for the formula $C_{21}H_{21}NO_4$ (351.41): | | | | | | |
|---|---|---|---|---|---|---|
| Calculated:<br>Found: | C: | 71.78<br>71.56 | H: | 6.02<br>6.00 | N: | 3.99%<br>4.05% |

$^1$H-NMR ($CDCl_3$): δ 8.42 (1H, d, J=5.5), 7.89 (1H, d, J=15.5), 7.68 (1H, d, J=15.5), 7.50 (1H, s), 7.40 (1H, d, J=5.5), 7.28 (1H, dd, J=8.3, 1.9), 7.20 (1H, d, J=1.9), 7.04 (1H, s), 6.91 (1H, d, J=8.3), 4.07 (3H,

s), 4.02 (3H, s), 3.96 (3H, s), 3.92 (3H, s).

Example 8

1-[2',4'-Di-(chloro)-styryl]-isoquinoline

According to the method of Example 1 1-[2',4'-di-(chloro)-styryl]-isoquinoline is prepared from 1-[methyl]-isoquinoline with 2,4-di-(chloro)-benzaldehyde. Yield 53%.
M.p.: 188 to 190 °C (dioxane)

| Analysis for the formula $C_{17}H_{11}Cl_2N$ (312.21): | | | | | | | | |
|---|---|---|---|---|---|---|---|---|
| Calculated: | C: | 69.25 | H: | 3.55 | N: | 4.49 | Cl: | 22.71% |
| Found: | | 68.04 | | 3.63 | | 4.61 | | 22.91%. |

$^1H$-NMR ($CDCl_3$): δ 8.59 (1H, d, J = 5.7), 8.33 (1H, d, J = 8.5), 8.26 (1H, d, J = 15.6), 7.95 (1H, d, J = 15.6), 7.80-7.20 (7H, m).

Example 9

1-[4'-(Cyano)-styryl]-3-[methyl]-6,7,8-tri-[methoxy]-isoquinoline

According to the method of Example 1 1-[4'-(cyano)-styryl]-3-[methyl]-6,7,8-tri-[methoxy]-isoquinoline is prepared from 1,3-di-[methyl]-6,7,8-tri-[methoxy]-isoquinoline with 4-(cyano)-benzaldehyde. Yield: 69%.
M.p.: 177 to 180.5 °C (acetone)

| Analysis for the formula $C_{22}H_{20}N_2O_3$ (360.42): | | | | | | |
|---|---|---|---|---|---|---|
| Calculated: | C: | 73.32 | H: | 5.59 | N: | 7.77% |
| Found: | | 72.36 | | 5.59 | | 7.99% |

$^1H$-NMR ($CDCl_3$): δ 8.61 (1H, d, J = 15.7), 7.73 (1H, d, J = 15.7), 7.72 (2H, d, J = 8.5), 7.64 (2H, d, J = 8.5), 7.29 (1H, s), 6.83 (1H, s), 4.00 (6H, s), 3.94 (3H, s), 2.67 (3H, s).

Example 10

1-[2'-(Fluoro)-styryl]-3-[methyl]-6,7-di-[methoxy]-isoquinoline

According to the method of Example 1 1-[2'-(fluoro)-styryl]-3-[methyl]-6,7-di-[methoxy]-isoquinoline is prepared from 1,3-di-[methyl]-6,7-di-[methoxy]-isoquinoline with 2-(fluoro)-benzalhyde. Yield: 54%.
M.p.: 138 to 139.5 °C (ethyl acetate)

| Analysis for the formula $C_{20}H_{18}FN0_2$ (323.37): | | | | | | | | |
|---|---|---|---|---|---|---|---|---|
| Calculated: | C: | 74.29 | H: | 5.61 | N: | 4.33 | F: | 5.87% |
| Found. | | 74.14 | | 5.56 | | 4.25 | | 6.02% |

$^1H$-NMR ($CDCl_3$) : δ 8.00 (1H, d, J = 15.8), 7.91 (1H, d, J = 15.8), 7.73 (1H, m), 7.45 (1H, s), 7.27 (1H, s), 7.35-7.10 (3H, m), 6.95 (1H, s) , 4.04 (3H, s), 4.00 (3H, s), 2.68 (3H, s).

Example 11

6,7-Di-[methoxy]-1-[3'-(methoxy)-styryl]-isoquinoline

According to the method of Example 1 6,7-di-[methoxy]-1-[3'-(methoxy)-styryl]-isoquinoline is prepared from 1-[methyl]-6,7-di-[methoxy]-isoquinoline with 3-(methoxy)-benzaldehyde.
Yield 54%.

M.p.: 129 to 130 ° C (methanol)

| Analysis for the formula $C_{20}H_{19}NO_3$ (321.39): | | | | | | |
|---|---|---|---|---|---|---|
| Calculated:<br>Found: | C: | 74.75<br>74.61 | H: | 5.96<br>5.80 | N: | 4.36%<br>4.39%. |

$^1H-NMR$ ($CDCl_3$): δ 8.42 (1H, d, J = 5.5), 7.91 (1H, d, J = 15.6), 7.79 (1H, d, J = 15.6), 7.47 (1H, s), 7.41 (1H, d, J = 5.5), 7.40 7.25 (3H, m), 7.03 (1H, s), 6.88 (1H, m), 4.06 (3H, s), 4.01 (3H, s), 3.86 (3H, s).

Example 12

6,7-Di-[methoxy]-1-[3',4',5'-tri(methoxy)-styryl]-isoquinoline

According to the method of Example 1 6,7-di-[methoxy]-1-[3',4',5'-tri-(methoxy)-styryl]-isoquinoline is prepared from 1-[methyl]-6,7-di-[methoxy]-isoquinoline with 3,4,5-tri-(methoxy)-benzaldehyde. Yield: 64%.
M.p. : 144 to 145.5 ° C (isopropanol)

| Analysis for the formula $C_{22}H_{23}NO_5$ (381.44): | | | | | | |
|---|---|---|---|---|---|---|
| Calculated:<br>Found: | C: | 69.28<br>67.70 | H: | 6.08<br>6.34 | N: | 3.67%<br>3.83% |

$^1H-NMR$ ($CDCl_3$) : δ 8.42 (1H, d, J = 5.5), 7.86 (1H, d, J = 15.5), 7.68 (1H, d, J = 15.5), 7.49 (1H, s), 7.42 (1H, d, J = 5.5), 7.06 (1H, s), 6.90 (2H, s), 4.07 (3H, s), 4.03 (3H, s), 3.94 (6H, s), 3.90 (3H, s).

Example 13

1-[3'-(Bromo)-styryl]-6,7-di-[methoxy]-isoquinoline

According to the method of Example 1 1-[3'-(bromo)-styryl]-6,7-di-[methoxy]-isoquinoline is prepared from 1-[methyl]-6,7-di-[methoxy]-isoquoinoline with 3-(bromo)-benzaldehyde.
Yield: 58%.
M.p.: 162 to 164 ° C (acetone)

| Analysis for the formula $C_{19}H_{16}BrNO_2$ (370.26): | | | | | | | | |
|---|---|---|---|---|---|---|---|---|
| Calculated:<br>Found: | C: | 61.64<br>61.27 | H: | 4.36<br>4.32 | N: | 3.78<br>3.71 | Br: | 21.58%<br>21.50% |

$^1H-NMR$ ($CDCl_3$) : δ 8.40 (1H, d, J = 5.5), 7.84 (1H, d, J = 15.5), 7.79 7.73 (1H, d, J = 15.5), (1H, t, J = 1.6), 7.60, 7.20 (3H, m), 7.40 7.29 (1H, d, J = 5.5), (1H, s), 6.99 (1H, s), 4.05 (3H, s), 3.98 (3H, s).

Example 14

6,7-Di-[methoxy]-1-[2'-(pyrid-2''-yl)-ethenyl]-isoquinoline

According to the method of Example 1 6,7-di-[methoxy]-1-[2'-(pyrid-2''-yl)-ethenyl]-isoquinoline is prepared from 1-[methyl]-6,7-di-[methoxy]-isoquinoline with pyrid-2-ylcarbaldehyde. Yield: 54%.
M.p.: 173 to 175 ° C (ethanol)

| Analysis for the formula $C_{18}H_{16}N_2O_2$ x $2H_2O$ (328.38): | | | | | | |
|---|---|---|---|---|---|---|
| Calculated:<br>Found: | C: | 65.84<br>66.17 | H: | 6.14<br>6.07 | N: | 8.53%<br>8.63% |

$^1$H-NMR ($CDCl_3$): δ 8.67 (1H, dd, J=4.60, 1.30), 8.53 (1H, d, J=15.1), 8.44 7.95 (1H, d, J=15.1), (1H, d, J= 5.5), (1H, d, J=5.5), 7.73-7.66 (1H, m), 7.65 (1H, s), 7.46-7.41 (1H, m), 7.44 7.23-7.17 (1H, m), 7.04 (1H, s), 4.09 (3H, s), 4.01 (3H, s).

Example 15

1-[2'-(Nitro)-styryl]-isoquinoline

According to the method of Example 1 1-[2'-(nitro)-styryl]-isoquinoline is prepared from 1-[methyl]-isoquinoline with 2-(nitro)-benzaldehyde. Yield: 51%.
M.p.: 156 to 160 °C (acetone)

| Analysis for the formula $C_{17}H_{12}N_2O_2$ (276.30): | | | | | | |
|---|---|---|---|---|---|---|
| Calculated:<br>Found: | C: | 73.90<br>73.44 | H: | 4.38<br>4.35 | N: | 10.14%<br>10.04% |

$^1$H-NMR ($CDCl_3$): δ 8.57 (1H, d, J=5.5), 8.33 (1H, d, J=15.3), 8.32 (1H, m), (7H, m) 7.89 (1H, d, J=15.3), 7.60 (1H, d, J=5.5), 8.00-7.50.

Example 16

6,7,8-Tri-[methoxy]-1-[3'-(nitro)-styryl]-isoquinoline

According to the method of Example 1 6,7,8-tri-[methoxy]-1-[3'-(nitro)-styryl]-isoquinoline is prepared from 1-[methyl]-6,7,8-tri-[methoxy]-isoquinoline with 3-(nitro)-benzaldehyde.
Yield: 56%.
M.p.: 149 to 154 °C (diisopropyl ether : ethanol = 5 : 1)

| Analysis for the formula $C_{20}H_{18}N_2O_5$ (366.38): | | | | | | |
|---|---|---|---|---|---|---|
| Calculated:<br>Found: | C: | 65.57<br>65.05 | H: | 4.95<br>4.90 | N: | 7.65%<br>7.70% |

$^1$H-NMR ($CDCl_3$): δ 8.64 (1H, d, J=15.7), 8.51 (1H, t, J=1.9), 8.43 (1H, d, J=5.6), 8.12 (1H, m), 7.93 (1H, d, J=7.9), 7.76 (1H, d, J=15.7), 7.55 (1H, t, J=7.9), 7.44 (1H, d, J=5.6), 6.93 (1H, s), 4.03 (3H, s), 4.01 (3H, s), 3.99 (3H, s).

Example 17

1-[4'(Bromo)-styryl]-6,7,8-tri-[methoxy]-isoquinoline

According to the method of Example 1 1-[4'-(bromo)-styryl]-6,7,8-tri-[methoxy]-isoquinoline is prepared from 1-[methyl]-6,7,8-tri-[methoxy]-isoquinoline with 4-(bromo)-benzaldehyde.
Yield: 56%.
M.p.: 115 to 117 °C (acetone : ethanol = 7 : 1)

| Analysis for the formula $C_{20}H_{18}BrNO_3$ (400.28): | | | | | | | | |
|---|---|---|---|---|---|---|---|---|
| Calculated:<br>Found: | C: | 60.01<br>59.84 | H: | 4.53<br>4.61 | N: | 3.49<br>3.32 | Br: | 19.96%<br>20.01% |

$^1$H-NMR ($CDCl_3$): δ 8.51 (1H, d, J=15.7), 8.41 (1H, d, J=5.5), 7.67 (1H, d, J=15.7), 7.52 (4H, m), 7.41 (1H, d, J=5.5), 6.91 (1H, s), 4.01 (6H, s), 3.94 (3H, s).

Example 18

3-[Methyl]-6,7-[methylenedioxy]-1-[2'-(thien-2''-yl)-ethenyl]-isoquinoline

According to the method of Example 1 3-[methyl]-6,7-[methylenedioxy]-1-[2'-(thien-2''-yl)-ethenyl]-isoquinoline is prepared from 1,3-di-[methyl]-6,7-[methylenedioxy]-isoquinoline with thiophen-2-carbaldehyde. Yield: 61%.
M.p.: 163 to 166 °C (ethyl acetate : n-heptane = 5 : 2)

| Analysis for the formula $C_{17}H_{13}NO_2S$ (295.37): | | | | | | | | |
|---|---|---|---|---|---|---|---|---|
| Calculated:<br>Found: | C: | 69.13<br>69.28 | H: | 4.44<br>4.53 | N: | 4.74<br>4.77 | S: | 10.86%,<br>10.85%. |

$^1$H-NMR (CDCl$_3$): δ 8.02 (1H, d, J = 15.3), 7.49 (1H, d, J = 15.3), 7.41 (1H, s), 7.25 (2H, m), 7.13 (1H, s), 7.05 (1H, m), 6.86 (1H, s), 5.98 (2H, s), 2.61 (3H, s).

Example 19

1-[3'(Bromo)-styryl]-3-[methyl]-6,7-[methylenedioxy]-isoquinoline

According to the method of Example 1 1-[3'-(bromo)-styryl]-3-[methyl]-6,7-[methylenedioxy]-isoquinoline is prepared from 1,3-di-[methyl]-6,7-[methylenedioxy]-isoquinoline with 3-(bromo)-benzaldehyde. Yield 53%.
M.p.: 189 to 190 °C (acetone)

| Analysis for the formula $C_{19}H_{14}BrNO_2$ (368.24): | | | | | | | | |
|---|---|---|---|---|---|---|---|---|
| Calculated:<br>Found: | C: | 61.97<br>61.70 | H: | 3.83<br>3.87 | N: | 3.80<br>3.84 | Br: | 21.70%,<br>21.32%. |

$^1$H-NMR (CDCl$_3$): δ 7.81 (1H, d, J = 15.5), 7.81 7.76 (1H, d, J = 15.5), (1H, m), 7.56-7.40 (3H, m), 7.28-7.21 (2H, m), 6.96 (1H, s), 6.07 (2H, s), 2.65 (3H, s).

Example 20

1-[3',4'-Di-(methoxy)-styryl]-6,7-[methylenedioxy]-isoquinoline

According to the method of Example 1 1-[3',4'-di-(methoxy)-styryl]-6,7-[methylenedioxy]-isoquinoline is prepared from 1-[methyl]-6,7-[methylenedioxy]-isoquinoline with 3,4-di-(methoxy)-benzaldehyde. Yield 55%.
M.p.: 204 to 207 °C (ethanol : dichloroethane = 6.5 : 1.0)

| Analysis for the formula $C_{20}H_{17}NO_4$ (335.37): | | | | | | |
|---|---|---|---|---|---|---|
| Calculated:<br>Found: | C: | 71.63<br>70.08 | H: | 5.11<br>5.24 | N: | 4.18%,<br>4.26%. |

$^1$H-NMR (CDCl$_3$): δ 8.40 (1H, d, J = 5.5), 7.87 (1H, d, J = 15.5), 7.64 (1H, d, J = 15.5), 7.62 (1H, s), 7.38 (1H, d, J = 5.5), 7.27-7.21 (2H, m), 7.08 (1H, s), 6.91 (1H, d, J = 8.0), 6.12 (2H, s), 3.98 (3H, s), 3.93 (3H, s).

Example 21

6,7-Di-[methoxy]-1-[4'-(methoxy)-styryl]-isoquinoline

According to the method of Example 1 6,7-di-[methoxy]-1-[4'-(methoxy)-styryl]-isoquinoline is prepared from 1-[methyl]-6,7-di-[methoxy]-isoquinoline with 4-(methoxy)-benzaldehyde.
Yield 52%.

M.p.: 146 to 150 ° C (acetone)

| Analysis for the formula $C_{20}H_{19}NO_3$ (321.39): | | | | | | |
|---|---|---|---|---|---|---|
| Calculated:<br>Found: | C: | 74.75<br>76.15 | H: | 5.96<br>5.89 | N: | 4.36%<br>4.43% |

$^1$H-NMR ($CDCl_3$): δ 8.41 (1H, d, J = 5.5), 7.90 (1H, d, J = 15.5), 7.70 (1H, d, J = 15.5), 7.63 (2H, d, J = 8.7), 7.50 (1H, s), 7.39 (1H, d, J = 5.5), 7.04 (1H, s), 6.95 (2H, d, 3 = 8.7), 4.07 (3H, s), 4.02 (3H, s), 3.84 (3H, s).

Example 22

1-[3',4'-Di-(methoxy)-styryl]-3-[methyl]-6,7-di-[methoxy]-isoquinoline

According to the method of Example 1 1-[3',4'-di-(methoxy)-styryl]-3-[methyl]-6,7-di-[methoxy]-isoquinoline is prepared from 1,3-di-[methyl]-6,7-di-[methoxy]-isoquinoline with 3,4-di-(methoxy)-benzaldehyde. Yield 68%.
M.p.: 139.5 to 140.5 ° C (acetone : water 1 : 1)

| Analysis for the formula $C_{22}H_{23}NO_4$ (365.44): | | | | | | |
|---|---|---|---|---|---|---|
| Calculated:<br>Found: | C: | 72.31<br>72.46 | H: | 6.31<br>6.45 | N: | 3.83%<br>3.68% |

$^1$H-NMR ($CDCl_3$): δ 7.86 (1H, d, J = 15.5), 7.66 (1H, d, J = 15.5), 7.47 (1H, s), 7.29-7.24 (2H, m), 7.20 (1H, d, J = 1.9), 6.95 (1H, s), 6.90 (1H, d, J = 8.3), 4.04 (3H, s), 4.00 (3H, s), 3.96 (3H, s), 3.91 (3H, s), 2.67 (3H, s).

Example 23

1-[3',4'-Di-(chloro)-styryl]-3-[methyl]-6,7-di-[methoxy]-isoquinoline

According to the method of Example 1 1-[3',4'-di-(chloro)-styryl]-3-[methyl]-6,7-di-[methoxy]-isoquinoline is prepared from 1,3-di-[methyl]-6,7-di-[methoxy]-isoquinoline with 3,4-di-(chloro)-benzaldehyde. Yield 51%.
M.p.: 154 to 156 ° C (acetone - water)

| Analysis for the formula $C_{20}H_{17}Cl_2NO_2$ (374.28): | | | | | | | | |
|---|---|---|---|---|---|---|---|---|
| Calculated:<br>Found: | C: | 64.19<br>64.96 | H: | 4.58<br>4.53 | N: | 3.74<br>3.65 | Cl: | 18.94%<br>19.07% |

$^1$H-NMR ($CDCl_3$): δ 7.83 (1H, d, J = 15.5), 7.74 (1H, d, J = 15.5), 7.73 (1H, s), 7.44 (2H, s), 7.40 (1H, s), 7.29 (1H, s) , 6.96 (1H, s), 4.06 (3H, s), 4.01 (3H, s), 2.67 (3H, s).

Example 24

1-[2'-(Bromo)-styryl]-6,7-di-[methoxy]-isoquinoline

According to the method of Example 1 1-[2'-(bromo)-styryl]-6,7-di-[methoxy]-isoquinoline is prepared from 1-[methyl]-6,7-di-[methoxy]-isoquinoline with 2-(bromo)-benzaldehyde.
Yield 69%.
M.p.: 181 to 182 ° C (acetone)

| Analysis for the formula $C_{19}H_{16}BrNO_2$ (370.26): | | | | | | | | |
|---|---|---|---|---|---|---|---|---|
| Calculated:<br>Found: | C: | 61.64<br>61.20 | H: | 4.36<br>4.29 | N: | 3.78<br>7.76 | Br: | 21.58%<br>21.39% |

$^1$H-NMR ($CDCl_3$): δ 8.44 (1H, d, J = 5.5), 8.18 (1H, d, J = 15.5), 7.85-7.70 (1H, d, J = 15.5), 7.15 (4H, m), 7.46 (1H, s), 7.40 (1H, d, J = 5.5), 7.01 (1H, s), 4.03 (3H, s), 3.99 (3H, s).

Example 25

6,7-[Methylenedioxy]-1-[3',4'(methylenedioxy)-styryl]-isoquinoline

According to the method of Example 1 6,7-[methylenedioxy]-1-[3',4'-(methylenedioxy)-styryl]-isoquinoline is prepared from 1-[methyl]-6,7-[methylenedioxy]-isoquinoline with 3,4-(methylenedioxy)-benzaldehyde. Yield 58%.
M.p.: 184 to 188 °C (ethylacetate : methanol = 4 : 1)

| Analysis for the formula $C_{19}H_{13}NO_4$ (319.33): | | | | | | |
|---|---|---|---|---|---|---|
| Calculated:<br>Found: | C: | 71.47<br>71.05 | H: | 4.10<br>4.13 | N: | 4.39 %,<br>4.37 %. |

$^1$H-NMR ($CDCl_3$): δ 8.38 (1H, d, J = 5.5), 7.83 (1H, d, J = 15.5), 7.59 (1H, d, J = 15.5), 7.56 (1H, s), 7.36 (1H, d, J = 5.5), 7.21 (1H, d, J = 1.4), 7.11 (1H, dd, J = 8.1, 1.4), 7.04 (1H, s), 6.83 (1H, d, J = 8.1), 6.08 (2H, s), 6.00 (2H, s).

Example 26

1-[2'-(Chloro)-styryl]-6,7,8-tri-[methoxy]-isoquinoline

According to the method of Example 1 1-[2'-(chloro)-styryl]-6,7,8-tri-[methoxy]-isoquinoline is prepared from 1-[methyl]-6,7,8-tri-[methoxy]-isoquinoline with 2-(chloro)-benzaldehyde. Yield 59%.
M.p. 120 to 123 °C (methanol)

| Analysis for the formula $C_{20}H_{18}ClNO_3$ (355.83): | | | | | | | | |
|---|---|---|---|---|---|---|---|---|
| Calculated:<br>Found: | C: | 67.51<br>67.25 | H: | 5.10<br>5.19 | N: | 3.94<br>3.98 | Cl: | 9.96%,<br>9.83%. |

$^1$H-NMR ($CDCl_3$): δ 8.49 (1H, d, J = 15.7), 8.44 (1H, d, J = 5.4), 8.12 (1H, d, J = 15.7), 7.85, (1H, d, J = 7.6, 1.8), 7.41 (1H, d, J = 1.6), 7.40 (1H, d, J = 5.4), 7.40-7.20 (2H, m), 6.90 (1H, s), 4.00 (3H, s), 3.99 (3H, s), 3.93 (3H, s).

Example 27

1-[4'-(Dimethylamino)-styryl]-3-[methyl]-6,7-[methylenedioxy]-isoquinoline

According to the method of Example 1 1-[4'-(dimethylamino)-styryl]-3-[methyl]-6,7-[methylenedioxy]-isoquinoline is prepared from 1,3-di-[methyl]-6,7-[methylenedioxy]-isoquinoline with 4-(dimethylamino)-benzaldehyde. Yield 51%.
M.p. : 183 to 187 °C (acetone)

| Analysis for the formula $C_{21}H_{20}N_2O_2$ (332.41): | | | | | | |
|---|---|---|---|---|---|---|
| Calculated:<br>Found: | C: | 75.88<br>75.70 | H: | 6.06<br>6.03 | N: | 8.43%<br>8.51% |

$^1$H-NMR ($CDCl_3$): δ 7.84 (1H, d, J=15.5), 7.56 (1H, d, J=15.5), 7.56 (2H, d, J=8.8), 7.55 (1H, s), 7.25 (1H, s), 6.94 (1H, s), 6.73 (2H, d, J=8.8), 6.05 (2H, s), 3.00 (6H, s), 2.65 (3H, s).

Example 28

1-[3'-(Chloro)-styryl]-6,7-di-[methoxy]-isoquinoline

According to the method of Example 1 1-[3'-(chloro)-styryl]-6,7-di-[methoxy]-isoquinoline is prepared from 1-[methyl]-6,7-di-[methoxy]-isoquinoline with 3-(chloro)-benzaldehyde.
Yield: 52%.
M.p.: 161 to 162°C (ethanol)

| Analysis for the formula $C_{19}H_{16}ClNO_2$ (325.80): | | | | | | | | |
|---|---|---|---|---|---|---|---|---|
| Calculated:<br>Found: | C: | 70.05<br>69.64 | H: | 4.95<br>4.82 | N: | 4.30<br>4.38 | Cl: | 10.88%<br>10.88% |

$^1$H-NMR ($CDCl_3$): δ 8.42 (1H, d, J=5.4), 7.88 (1H, d, J=15.5), 7.78 (1H, d, J=15.5), 7.65 (1H, s) , 7.55-7.25 (5H, m), 7.04 (1H, s), 4.08 (3H, s), 4.01 (3H, s).

II) Method B

Example 29

1-[3'-(Fluoro)-styryl]-isoquinoline

A mixture of 6.44 g (0.045 mole) of 1-[methyl]-isoquinoline, 6.2 g (0.05 mole) of 3-(fluoro)-benzaldehyde and 18 ml of acetic acid is stirred at a temperature of from 130 to 140°C for 3 hours. The termination of the reaction is controlled by thin layer chromatography. The reaction mixture is cooled and 100 ml of anhydrous acetone are added to it. Then it is acidified with isopropanol saturated with dry hydrogen chloride gas and isopropanol until pH = 1 under stirring and cooling with ice-water. The suspension is stirred at a temperature of from 0 to 5°C for 2 hours. The separated hydrochloride salt is filtered, washed with cold acetone and dissolved in 180 ml of water. To the thus obtained mixture 20% by weight sodium hydroxide solution is dropped until pH = 11 to 12 under stirring and cooling with ice-water, and the solution is extracted three times with 75 ml each of dichloromethane. The extracts are combined, washed with an aqueous sodium chloride solution, dried over $Na_2SO_4$ and evaporated. The crude product is recrystallized from 30 ml of isopropanol. Thus 8.7 g (78%) of 1-[3'-(fluoro)-styryl]-isoquinoline are obtained.
M.p.: 89 to 99°C (isopropanol)

| Analysis for the formula $C_{17}H_{12}FN$ (249.30): | | | | | | |
|---|---|---|---|---|---|---|
| Calculated:<br>Found: | C: | 81.91<br>81.45 | H: | 4.85<br>4.94 | N: | 5.62%<br>5.72% |

$^1$H-NMR ($CDCl_3$) : δ 8.55 (1H, d, J=5.6), 8.33 (1H, d, J=8.2), 7.99 (1H, d, J=16.0), 7.91 (1H, d, J=16.0), 7.80-6.95 (8H, m).

Example 30

1-[2'-(Fur-2''-yl)-ethenyl]-6,7-di-[methoxy]-isoquinoline

According to the method of Example 29 1-[2'-(fur-2''-yl)-ethenyl]-6,7-di-[methoxy]-isoquinoline is prepared from 1-[methyl]-6,7-di-[methoxy]-isoquinoline and (fur-2''-yl)-carbaldehyde.
Yield: 51%.
M.p.: 185 to 190 ° C (ethanol : water = 9 : 1)

| Analysis for the formula $C_{17}H_{15}N0_3$ (281.32): | | | | | | |
|---|---|---|---|---|---|---|
| Calculated:<br>Found: | C: | 72.58,<br>72.87, | H: | 5.37,<br>5.29, | N: | 4.98 %<br>5.10 %. |

$^1$H-NMR ($CDCl_3$) : δ 8.37 (1H, d, J=5.5), 7.78 (1H, d, J=15.3), 7.71 (1H, d, J=15.3), 7.47 (1H, d, J=1.6), 7.43 (1H, s), 7.34 (1H, d, J= 5.5), 6.96 (1H, s), 6.55-6.45 (2H, m), 4.04 (3H, s), 3.97 (3H, s).

Example 31

1-[4'-(Hydroxy)-3'-(methoxy)-styryl]-isoquinoline

According to the method of Example 29 1-[4'-(hydroxy)-3'-(methoxy)-styryl]-isoquinoline is prepared from 1-[methyl]-isoquinoline with 3-(methoxy)-4-(hydroxy)-benzaldehyde with the exception that the liberation of the free base has been omitted.
Yield: 50%.
M.p. : 190 to 230 ° C [decomp.] (ethanol : water = 10 : 1)

| Analysis for the formula $C_{18}H_{15}NO_2$ x HCl (313.79): | | | | | | | | |
|---|---|---|---|---|---|---|---|---|
| Calculated:<br>Found: | C: | 68.90<br>66.34 | H: | 5.14<br>5.35 | N: | 4.46<br>4.56 | Cl: | 11.30%<br>11.03% |

$^1$H-NMR (DMSO): δ 9.06 (1H, d, J=8.6), 8.46-8.39 (2H, m), 8.27-8.00 (5H, m), 7.64 (1H, d, J=1.7), 7.30 (1H, m), 6.97 (1H, d, J=8.1), 3.95 (3H, s).

Example 32

[3',4'-(Methylenedioxy)-styryl]-6,7-di-[methoxy]-isoquinoline

According to the method of Example 1 [3',4'-(methylenedioxy)-styryl]-6,7-di-[methoxy]-isoquinoline is prepared from 1-[methyl]-6,7-di-[methoxy]-isoquinoline and 3,4-(methylenedioxy)-benzaldehyde. Yield: 63%.
M.p.: 164 to 166 ° C (methanol)

| Analysis for the formula: $C_{20}H_{17}NO_4$ (335.37): | | | | | | |
|---|---|---|---|---|---|---|
| Calculated:<br>Found: | C: | 71.63,<br>71.41, | H: | 5.11,<br>5.07, | N: | 4.18%,<br>4.14%. |

$^1$H-NMR ($CDCl_3$): δ 8.38 (1H, d, J=5.4), 7.85 (1H, d, J=15.4), 7.61 (1H, d, J=15.4), 7.41 (1H, s), 7.35 (1H, d, J=5.5), 7.21 (1H, d, J=1.6), 7.10 (1H, dd, J=8.1, 1.6), 6.98 (1H, s), 6.82 (1H, d, J=8.1), 5.97 (2H, s), 4.03 (3H, s), 3.98 (3H, s).

Example 33

3-[Methyl]-6,7,8-tri-[methoxy]-1-[2'-(pyrid-3''-yl)-ethenyl]-isoquinoline

According to the method of Example 1 3-[methyl]-6,7,8-tri-[methoxy]-1-[2'-(pyrid-3''-yl)-ethenyl]-isoquinoline is prepared from 1,3-di-[methyl]-6,7,8-tri-[methoxy]-isoquinoline with pyrid-3-ylcarbaldehyde. Yield: 51%.
M.p.: 93 to 95 ° C (diisopropyl ether)

| Analysis for the formula $C_{20}H_{20}N_2O_3$ (336.40): | | | | | | |
|---|---|---|---|---|---|---|
| Calculated:<br>Found: | C: | 71.41<br>71.32 | H: | 5.99<br>6.00 | N: | 8.33%<br>8.27% |

$^1H$-NMR ($CDCl_3$): δ 8.90 (1H, d, J = 1.9), 8.58 (1H, d, J = 15.7), 8.51 (1H, dd, J = 4.7, 1.4), 7.96 (1H, d, J = 8.0), 7.73 (1H, d, J = 15.7), 7.30 (1H, dd, J = 7.9, 4.8), 7.25 (1H, s), 6.80 (1H, s), 3.99 (3H, s), 3.97 (3H, s), 3.95 (3H, s), 2.67 (3H, s).

Example 34

1-[2',5'-Di-(methoxy)-styryl]-3-[methyl]-6,7,8-tri-[methoxy]-isoquinoline

According to the method of Example 1 1-[2',5'-di-(methoxy)-styryl]-3-[methyl]-6,7,8-tri-[methoxy]-isoquinoline is prepared from 1,3-di-[methyl]-6,7,8-tri-[methoxy]-isoquinoline with 2,5-di-(methoxy)-benzaldehyde.
Yield: 54%.
M.p.: 102 to 110 ° C (isopropanol)

| Analysis for the formula $C_{23}H_{25}NO_5$ (395.47): | | | | | | |
|---|---|---|---|---|---|---|
| Calculated:<br>Found: | C: | 69.86<br>69.79 | H: | 6.37<br>6.33 | N: | 3.54%<br>3.63% |

$^1H$-NMR ($CDCl_3$): δ 8.53 (1H, d, J = 15.8), 8.13 (1H, d, J = 15.8), 7.31 (1H, d, J = 2.4), 7.19 (1H, s), 6.83 (2H, m), 6.76 (1H, s), 3.97 (3H, s), 3.95 (3H, s), 3.93 (3H, s), 3.91 (3H, s), 3.90 (3H, s), 2.66 (3H, s).

Example 35

3-[Methyl]-6,7,8-tri-[methoxy]-1-[2'-(naphth-1''-yl)-ethenyl]-isoquinoline

According to the method of Example 1 3-[methyl]-6,7,8-tri-[methoxy]-1-[2'-(naphth-1''-yl)-ethenyl] -isoquinoline is prepared from 1,3-di-[methyl]-6,7,8-tri- [methoxy]-isoquinoline with naphth-1-yl-aldehyde Yield: 54%.
M.p.: 99 to 106.5 ° C (methanol)

| Analysis for the formula $C_{25}H_{23}NO_3$ (385.48): | | | | | | |
|---|---|---|---|---|---|---|
| Calculated:<br>Found: | C: | 77.90<br>79.29 | H: | 6.01<br>6.32 | N: | 3.63%<br>3.81% |

$^1H$-NMR ($CDCl_3$): δ 8.62 (1H, d, J = 15.3), 8.56 (1H, d, J = 15.3), 8.43 (1H, d, J = 7.3), 7.96 (1H, d, J = 6.6), 7.80(2H, m), 7.50 (3H, m), 7.21 (1H, s), 6.75 (1H, s), 3.96 (3H, s), 3.91, 3.90 (3H, s), (3H, s), 2.70 (3H, s).

Example 36

3-[Methyl]-1-[3',4'-(methylenedioxy)-2'-(methoxy)-styryl]-6,7,8-tri-[methoxy]-isoquinoline

According to the method of Example 1 3-[methyl]-1-[3',4'-(methylenedioxy)-2'-(methoxy)-styryl]-6,7,8-tri-[methoxy]-isoquinoline is prepared from 1,3-di-[methyl]-6,7,8-tri-[methoxy]-isoquinoline with 2-(methoxy)-3,4-(methylenedioxy)-benzaldehyde. Yield: 51%.
M.p.: 110 to 115 °C (n-heptane : ethyl acetate = 12 : 5)

| Analysis for the formula $C_{23}H_{23}NO_6$ (409.45): | | | | | | |
|---|---|---|---|---|---|---|
| Calculated:<br>Found: | C: | 67.47<br>67.00 | H: | 5.66<br>5.70 | N: | 3.42%<br>3.49% |

$^1$H-NMR ($CDCl_3$): δ 8.45 (1H, d, J=15.7), 7.92 (1H, d, J=15.7), 7.25 (1H, d, J=8.2), 7.19 (1H, s), 6.78 (1H, s), 6.59 (1H, d, J=8.2), 5.96 (2H, s), 4.08 (3H, s), 3.97 (3H, s), 3.96 (3H, s), 3.92 (3H, s), 2.65 (3H, s).

Example 37

6,7,8-Tri-[methoxy]-1-[styryl]-isoquinoline

According to the method of Example 1 6,7,8-tri-[methoxy]-1-[styryl]-isoquinoline is prepared from 1-[methyl]-6,7,8-tri-[methoxy]-isoquinoline with benzaldehyde. Yield: 53%.
M.p.: 86 to 88.5 °C (water : ethanol = 3 : 1)

| Analysis for the formula $C_{20}H_{19}NO_3$ (321.38): | | | | | | |
|---|---|---|---|---|---|---|
| Calculated:<br>Found: | C: | 74.75<br>74.60 | H: | 5.96<br>5.93 | N: | 4.36%<br>4.38% |

$^1$H-NMR ($CDCl_3$): δ 8.54 (1H, d, J=15.7), 8.42 (1H, d, J=5.5), 7.76 (1H, d, J=15.7), 7.75-7.25 (5H, m), 7.39 (1H, d, J=5.5), 6.90 (1H, s), 4.01 (3H, s), 3.99 (3H, s), 3.95 (3H, s).

Example 38

6,7,8-Tri-[methoxy]-1-[3,4,5-tri-(methoxy)-styryl]-isoquinoline

According to the method of Example 1 6,7,8-tri-[methoxy]-1-[3,4,5-tri-(methoxy)-styryl]-isoquinoline is prepared from 1-[methyl]-6,7,8-tri-[methoxy]-isoquinoline with 3,4,5-tri-(methoxy)-benzaldehyde. Yield 51%.
M.p.: 117.5 to 125.5 °C (water : ethanol = 4 : 1.5)

| Analysis for the formula $C_{23}H_{25}NO_6$ (411.47): | | | | | | |
|---|---|---|---|---|---|---|
| Calculated:<br>Found: | C: | 67.14<br>66.95 | H: | 6.12<br>6.05 | N: | 3.40%<br>3.47% |

$^1$H-NMR ($CDCl_3$): δ 8.45 (1H, d, J=15.6), 8.42, 7.69 (1H, d, J=15.6), (1H, d, J=5.6), 7.39 (1H, d, J=5.6), 6.93 (2H, s), 6.92 (1H, s), 4.02 (6H, s), 3.98 (3H, s), 3.93 (6H, s), 3.89 (3H, s).

Example 39

6 parts by weight of 6,7-[methylenedioxy]-1-[4'-(trifluoromethyl)-styryl]-isoquinoline {compound of Example 3} are admixed with 9 parts by weight of lactose and 3 parts by weight of microcrystalline cellulose. The powder mixture thus obtained is granulated with a solution of 0.5 parts by weight of polyvinyl pyrrolidone and 4 parts by weight of ion-exchanged water in a fluidization-spraying apparatus. The granules are dried, whereupon 1.3 parts by weight of sodium carboxymethyl cellulose and 0.2 part by weight of magnesium stearate are added. The granules are sieved through a sieve (mesh size: 1.0 mm). The sieved

granulate is pressed into tablets weighing 200 mg and having an active ingredient content of 60 mg, or filled into hard gelatine capsules size No. 2.

Example 40

10 parts by weight of 6,7-[methylenedioxy]-1-[4'-(trifluoromethyl)-styryl]-isoquinoline {compound of Example 3} are admixed with 9 parts by weight of hydroxypropylmethyl cellulose (Methocel® K 4 M) and 10 parts by weight of lactose. The powder mixture is granulated with the solution of 0.4 part by weight of polyvinyl pyrrolidone and 4 parts by weight of isopropanol in a whirlpool type granulating apparatus. The granules are dried. To the dried granules 0.3 part of weight of talc and 0.3 part by weight of magnesium stearate are added. The granules are sieved through a sieve (mesh size 1.0 mm). The sieved granulate is pressed into sustained release tablets weighing 300 mg and having an active ingredient content of 100 mg.

Example 41

1 part by weight of 6,7-[methylenedioxy]-1-[4'-(trifluoromethyl)-styryl]-isoquinoline {compound of Example 3} is dispersed in a melt of 599 parts by weight of a suppository base on the basis of modified saturated fatty acids of vegetable origin [Witepsol® S 58] at 50 °C. The liquid suspension is poured into suppository forms, allowed to solidify by cooling to 25 °C, whereupon the suppositories are removed. Thus suppositories weighing 6 g and having an active ingredient content of 10 mg are prepared.

**Claims**

**1.** 1-[Styryl]- and 1-[2'-(heteroaryl)-ethenyl] -isoquinoline derivatives having the formula

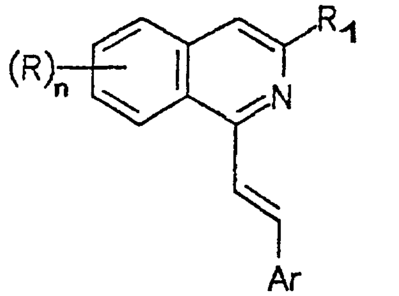

I )

wherein

n is 1, 2, 3 or 4,

R which may be identical or different, represent(s) hydrogen, $C_{1-7}$-alkyl, $C_{1-7}$-alkoxy or hydroxy, or two substituents R form together a $C_{1-7}$-alkylenedioxy group, attached to two adjacent ring carbon atoms,

$R_1$ represents hydrogen or $C_{1-7}$-alkyl and

Ar stands for an aryl or heteroaryl group containing one or two oxygen, nitrogen and/or sulfur atom(s), optionally carrying one or more identical or different substituent(s) selected from the group consisting of halogen, trihalomethyl, hydroxy, $C_{1-7}$-alkyl, $C_{1-7}$-alkoxy, $C_{1-7}$-alkylenedioxy (attached to two adjacent ring carbon atoms), cyano, nitro, amino, mono- and di-($C_{1-7}$-alkyl)-amino,

as well as acid addition salts thereof,

with the further provision that

6,7-di-[methoxy]-1-[3',4'-di-(methoxy)-styryl]-isoquinoline,
6,7-di-[methoxy]-1-[3',4'-(methylenedioxy)-styryl]-isoquinoline,
6,7-di-[methoxy]-1-[3',4',5'-tri-(methoxy)-styryl]-isoquinoline,
6,7-di-[methoxy]-1-[3'-(methoxy)-4'-(hydroxy)-styryl]-isoquinoline,
6,7-di-[methoxy]-1-[3'-(hydroxy)-4'-(methoxy)-styryl]-isoquinoline,
6,7-di-[methoxy]-1-[4'-(chloro)-styryl]-isoquinoline,
6,7-di-[methoxy]-1-[styryl]-isoquinoline,
6,7-[methylenedioxy]-1-[3',4'-di-(methoxy)-styryl]-isoquinoline,
6,7-[methylenedioxy]-1-[3',4'-(methylenedioxy)-styryl]-isoquinoline,

6,7-di-[methoxy]-3-[methyl]-1-[4'-(chloro)-styryl]-isoquinoline,
6,7-di-[methoxy]-3-[methyl]-1-[3',4'-di-(methoxy)-styryl]-isoquinoline,
6,7-di-[methoxy]-3-[methyl]-1-[3',4'-(methylenedioxy)-styryl]-isoquinoline,
6,7-di-[methoxy]-3-[methyl]-1-[2'-(pyrid-3''-yl)-ethenyl]-isoquinoline, and
6,7-di-[methoxy]-1-[2'-(pyrid-3''-yl)-ethenyl]--isoquinoline
as well as their acid addition salts
are excepted.

**2.** 1-[Styryl]- and 1-[2'-(heteroaryl)-ethenyl]-isoquinoline derivatives according to claim 1, characterized in that the alkyl group(s) which may be represented by R and/or $R_1$ and/or the alkoxy group(s) which may be represented by R and/or the alkyl and/or alkoxy group(s) by which the aryl or heteroaryl group which is represented by Ar may be substituted and/or the alkyl part(s) of the mono- and/or di-($C_{1-7}$-alkyl)-amino group(s) by which the aryl or heteroaryl group which is represented by Ar my be substituted is/are such having from 1 to 4 carbon atom(s).

**3.** 1-[Styryl]- and 1-[2'-(heteroaryl)-ethenyl]-isoquinoline derivatives according to claim 1 or 2, characterized in that the alkylenedioxy group (attached to two adjacent ring carbon atoms) which may be formed by two R and/or the alkylenedioxy group (attached to two adjacent ring carbon atoms) by which the aryl or heteroaryl group which is represented by Ar may be substituted is/are such having from 1 to 4 carbon atom(s) .

**4.** 1-[Styryl]- and 1-[2'-(heteroaryl)-ethenyl]-isoquinoline derivatives according to claims 1 to 3, characterized in that the halogen atom(s) and/or the halogen atoms of the trihalomethyl group(s) by which the aryl or heteroaryl group which is represented by Ar may be substituted is/are [a] chlorine and/or fluorine atom(s).

**5.** 1-[Styryl]- and 1-[2'-(heteroaryl)-ethenyl]-isoquinoline derivatives according to claims 1 to 4, characterized in that the aryl group which my be represented by Ar is a phenyl or naphthyl group.

**6.** 1-[Styryl]- and 1-[2'-(heteroaryl)-ethenyl]-isoquinoline derivatives according to claims 1 to 4, characterized in that the heteroaryl group which may be represented by Ar is a furyl, thienyl, pyrrolyl, pyridyl, pyrazinyl, pyridazinyl, oxazolyl, isoxazolyl, thiazolyl, isothiazolyl, pyrimidyl, quinolyl or isoquinolyl group.

**7.** 1-[Styryl]- and 1-[2'-(heteroaryl)-ethenyl]-isoquinoline derivatives according to claims 1 to 6, characterized in that they have the formula

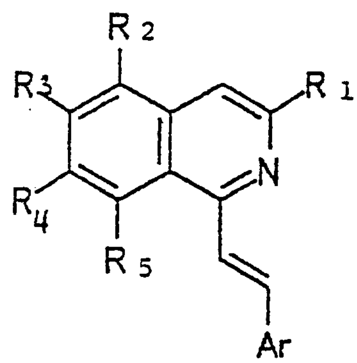

Ia ,

wherein

| | |
|---|---|
| $R_1$ | stands for hydrogen or $C_{1-7}$-alkyl, |
| $R_2$ | represents hydrogen, |
| $R_3$ and $R_4$ | each represent hydrogen or $C_{1-7}$-alkoxy or together form a $C_{1-7}$-alkylenedioxy group, |
| $R_5$ | represents hydrogen or $C_{1-7}$-alkoxy and |
| Ar | denotes phenyl, optionally carrying one or more identical or different substituent(s) selected from the group consisting of halogen, trihalomethyl, hydroxy, $C_{1-7}$-alkyl, |

$C_{1-7}$-alkoxy, $C_{1-7}$-alkylenedioxy (attached to two adjacent ring carbon atoms), cyano, nitro, amino, mono- and di-($C_{1-7}$-alkyl)-amino, or naphthyl, optionally carrying the above-mentioned substituents, or mono- or bicyclic heteroaryl containing one or two oxygen, nitrogen and/or sulfur atom(s) and, optionally carrying the above substituents

as well as their acid addition salts.

**8.** 1-[Styryl]- and 1-[2'-(heteroaryl)-ethenyl]-isoquinoline derivatives according to claim 7, characterized in that

$R_1$ stands for hydrogen or methyl,
$R_2$ represents hydrogen,
$R_3$ and $R_4$ each represent methoxy or together form, a methylenedioxy group (attached to two adjacent ring carbon atoms),
$R_5$ denotes hydrogen or methoxy and
Ar stands for phenyl, carrying a substituent selected from the group consisting of halogen, trifluoromethyl, nitro, methoxy, cyano and methylenedioxy (attached to two adjacent ring carbon atoms).

**9.** 1-[Styryl]- and 1-[2'-(heteroaryl)-ethenyl]-isoquinoline derivatives according to claim 7 or 8, characterized in that

Ar represents phenyl, carrying a substituent in position 4 or having one substituent each in the positions 3 and 4 or bearing methylenedioxy in positions 3 and 4.

**10.** 1-[Styryl]- and 1-[2'-(heteroaryl)-ethenyl]-isoquinoline derivatives according to claims 7 to 9, characterized in that

$R_1$ and $R_2$ stand for hydrogen,
$R_3$ and $R_4$ each represent methoxy or together form, a methylenedioxy group attached in positions 3 and 4,
$R_5$ denotes hydrogen and
Ar stands for 4-(trifluoromethyl)-phenyl, 4-(fluoro)-phenyl or 3,4-di-(methoxy)-phenyl.

**11.** 6,7-[Methylenedioxy]-1-[4'-(trifluoromethyl)-styryl]-isoquinoline, 1-[4'-(fluoro)-styryl]-6,7-di-[methoxy]-isoquinoline, 3-[methyl]-6,7-di-[methoxy]-1-[4'-(nitro)-styryl]-isoquinoline, 6,7-di-[methoxy]-1-[4'-(trifluoromethyl)-styryl]-isoquinoline, 1-[4'-(cyano)-styryl]-3-[methyl]-6,7,8-tri-[methoxy]-isoquinoline and 6,7-di-[methoxy]-1-[4'-(methoxy)-styryl]-isoquinoline as well as acid addition salts thereof.

**12.** Process for preparing the 1-[styryl]- and 1-[2'-(heteroaryl)-ethenyl]-isoquinoline derivatives according to claims 1 to 11, characterized in that in a manner known per se 1-[methyl]-isoquinoline derivatives of general formula

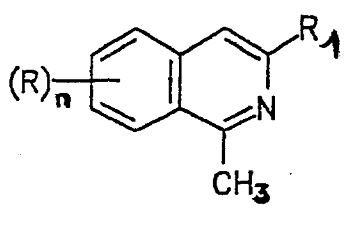

II,

wherein

n, R and $R_1$ are as defined in claims 1 to 3 or 7, 8 or 10, are reacted with aldehydes of general formula

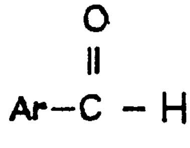

III,

wherein Ar is as defined in claims 1 to 10, in the presence of a condensing agent or an acidic catalyst and optionally 1-[styryl]- and 1-[2'-(heteroaryl)-ethenyl]-isoquinoline derivatives of general formula I thus obtained are converted into acid addition salts thereof or acid addition salts of 1-[styryl]- and 1-[2'-(heteroaryl)-ethenyl]-isoquinoline derivatives of general formula I thus obtained are converted into the free isoquinoline derivative bases of general formula I and/or into other acid addition salts.

**13.** Process according to claim 12 for preparing the 1-[styryl]-and 1-[2'-(heteroaryl)-ethenyl]-isoquinoline derivatives of claims 7 to 11, characterized in that as 1-[methyl]-isoquinoline derivatives of general formula II such of general formula

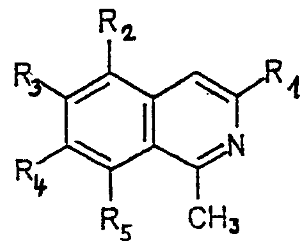

IIa,

wherein

$R_1$, $R_2$, $R_3$, $R_4$ and $R_5$ are as defined in claims 7 to 10,

are reacted with the aldehydes of general formula III, in which Ar is as defined in claims 1 to 10.

**14.** Process according to claim 12 or 13, characterized in that as condensing agent(s) zinc chloride, phosphorus pentoxide, acetic anhydride and/or propionic anhydride is/are used.

**15.** Process according to claim 12 or 13, characterized in that as acidic catalyst(s) [a] $C_{1-7}$-monocarboxylic acid(s), above all acetic acid, is/are used.

**16.** Medicaments, characterized in that they contain as [an] active ingredient(s) at least one compound according to claims 1 to 11, suitably in admixture with at least one suitable inert solid and/or liquid pharmaceutical carrier and/or diluent and/or excipient.

**17.** Use of the compounds according to claims 1 to 11 and/or of 6,7-di-[methoxy]-1-[3',4'-di-(methoxy)-styryl]-isoquinoline, 6,7-di-[methoxy]-1-[3',4'-(methylenedioxy)-styryl]-isoquinoline, 6,7-di-[methoxy]-1-[3',4',5'-tri-(methoxy)-styryl]-isoquinoline, 6,7-di-[methoxy]-1-[3'-(methoxy)-4'-(hydroxy)-styryl]-isoquinoline,6,7-di-[methoxy]-1-[3'-(hydroxy)-4'-(methoxy)-styryl]-isoquinoline,6,7-di-[methoxy]-1-[4'-(chloro)-styryl]-isoquinoline, 6,7-di-[methoxy]-1-[styryl]-isoquinoline, 6,7-[methylenedioxy]-1-[3',4'-di-(methoxy)-styryl]-isoquinoline, 6,7-[methylenedioxy]-1-[3',4'-(methylenedioxy)-styryl]-isoquinoline, 6,7-di-[methoxy]-3-[methyl]-1-[4'-(chloro)-styryl]-isoquinoline, 6,7-di-[methoxy]-3-[methyl]-1-[3',4'-di-(methoxy)-styryl]-isoquinoline, 6,7-di-[methoxy]-3-[methyl]-1-[3',4'-(methylenedioxy)-styryl]-isoquinoline, and/or 6,7-di-[methoxy]-1-[2'-(pyrid-3''-yl)-ethenyl]-isoquinoline and/or of their acid addition salts for preparing medicaments having an anxiolytic effect.

European Patent Office

# EUROPEAN SEARCH REPORT

Application Number
EP 95 10 6463

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | DE-A-19 02 402 (VEB ARZNEIMITTELWERK,DRESDEN)<br>* the whole document *<br>--- | 1,2, 4-10, 12-17 | C07D217/14<br>A61K31/47<br>C07D401/06<br>C07D217/16<br>C07D405/06<br>C07D217/02<br>C07D491/04 |
| X | JOURNAL OF HETEROCYCLIC CHEMISTRY, vol. 7, no. 6, December 1970 PROVO US, pages 1421-1423, LOUIS AMOROS-MARIN ET AL 'Derivatives of dibenzo[a,f]quinolizinium ion'<br>* the whole document *<br>--- | 1,2,4,5, 7 | |
| X | JOURNAL OF ORGANIC CHEMISTRY, vol. 31, no. 11, November 1966 EASTON US, pages 3683-3685, ALAN FOZARD ET AL 'New tetracyclic systems incorporating the benzo[c]quinolizinium cation'<br>* the whole document *<br>--- | 1,2,4,5, 7,8 | |
| X | DE-C-576 532 (C.H.BOEHRINGER SOHN)<br>* the whole document *<br>--- | 1,2,5,7, 16,17 | TECHNICAL FIELDS SEARCHED (Int.Cl.6)<br>C07D<br>A61K |
| X | ARZNEIMITTEL FORSCHUNG DRUG RESEARCH., vol. 31, no. 3, 1981 AULENDORF DE, pages 404-406, C.T. BAHNER ET AL 'Di-and tri-methoxystyryl derivatives of heterocyclic nitrogen compounds'<br>* the whole document *<br>---<br>-/-- | 1,2,5, 7-10,16, 17 | |

The present search report has been drawn up for all claims

| Place of search | Date of completion of the search | Examiner |
|---|---|---|
| THE HAGUE | 21 June 1995 | Henry, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application Number
EP 95 10 6463

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | JOURNAL OF ORGANIC CHEMISTRY, vol. 22, no. 6, 12 June 1957 EASTON US, pages 683-684, CARL TABB BAHNER ET AL 'Isoquinoline analogs of 4-(p-dimethylaminostyryl)quinoline' * the whole document * --- | 1,5,7,9, 16 | |
| X | JOURNAL OF THE AMERICAN CHEMICAL SOCIETY, vol. 94, no. 8, 19 April 1972 DC US, pages 2874-2875, EDWARD C. TAYLOR ET AL 'A facile method for alkylation and alkenylation of heterocycles' * the whole document * --- | 1,5,7,12 | |
| X | CHEMICAL ABSTRACTS, vol. 72, no. 7, 16 February 1970 Columbus, Ohio, US; abstract no. 29987z, FRITZ MARKWARDT ET AL 'Influence of 6,7-dimethoxyisoquinoline derivatives on the function of thrombocytes' page 153; column 2; * abstract * & ACTA BIOL. MED.GER, vol. 23, no. 2, 1969 pages 295-306, --- | 1,4-7,16 | TECHNICAL FIELDS SEARCHED (Int.Cl.6) |
| | -/-- | | |

The present search report has been drawn up for all claims

| Place of search | Date of completion of the search | Examiner |
|---|---|---|
| THE HAGUE | 21 June 1995 | Henry, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number
EP 95 10 6463

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | CHEMICAL ABSTRACTS, vol. 73, no. 3, 20 July 1970 Columbus, Ohio, US; abstract no. 14639j, JOACHIM KNABE ET AL 'Dihydroisoquinoline rearrangement' page 347; column 1; * abstract * & ARCH. PHARM (WEINHEIM), vol. 303, no. 5, 1970 pages 404-412, --- | 1,2,5,7 | |
| X | CHEMICAL ABSTRACTS, vol. 55, no. 14, 10 July 1961 Columbus, Ohio, US; abstract no. 13428b, N. ADITYA CHAUDHURY ET AL 'Beta-phenylethanolamines' column 13428b; * abstract * & J INDIAN CHEM. SOC., vol. 38, 1961 page 15-18 --- | 1,3,5,8 | TECHNICAL FIELDS SEARCHED (Int.Cl.6) |
| X | CHEMICAL ABSTRACTS, vol. 67, no. 21, 20 November 1967 Columbus, Ohio, US; abstract no. 97907v, KATSURO FUJIMOTO 'Studies on the relation between chemical structure and antimicrobial action of nitrofuran derivatives' page 9198; column 1; * abstract * & NIPPON KAGAKU RYOHOGAKUKAI ZASSHI, vol. 15, no. 3, 1967 pages 228-245, --- | 1,6,16 | |
| | -/-- | | |

The present search report has been drawn up for all claims

| Place of search | Date of completion of the search | Examiner |
|---|---|---|
| THE HAGUE | 21 June 1995 | Henry, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number
EP 95 10 6463

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | CHEMICAL ABSTRACTS, vol. 72, no. 3, 19 January 1970 Columbus, Ohio, US; abstract no. 11880z, JERZY CHODKOWSKI ET AL 'Polarographic behavior of isomeric pyridylvinylquinolines and isoquinolines' page 255; column 1; * abstract * & ROCZ.CHEM., vol. 43, no. 5, 1969 pages 1037-1051, ----- | 1,6 | |

TECHNICAL FIELDS SEARCHED (Int.Cl.6)

The present search report has been drawn up for all claims

| Place of search | Date of completion of the search | Examiner |
|---|---|---|
| THE HAGUE | 21 June 1995 | Henry, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)